# EUROPÄISCHE PATENTSCHRIFT

(11) **EP 4 034 260 B1**
(45) Veröffentlichungstag und Bekanntmachung des Hinweises auf die Patenterteilung: **14.01.2026**
(21) Anmeldenummer: 20764984.9
(22) Anmeldetag: 31.08.2020
(51) Int. Cl.: A61Q 5/06, A61K 8/86, A61K 8/898, A61K 8/31, A61K 8/37, A61K 8/34

(54) **VERFAHREN ZUM FÄRBEN VON KERATINISCHEM MATERIAL MIT PREMIX AUS AMINOSILIKON UND FARBGEBENDER VERBINDUNG**
METHOD FOR DYEING KERATINOUS MATERIAL BY MEANS OF A PREMIX OF AMINOSILICONE AND A CHROMOPHORIC COMPOUND
PROCÉDÉ DE COLORATION DE MATIÈRES KÉRATINIQUES AU MOYEN D'UN PRÉMÉLANGE D'AMINOSILICONE ET D'UN COMPOSÉ CHROMOPHORE

(30) Priorität: 23.09.2019 DE 102019214463
(43) Veröffentlichungstag der Anmeldung: 03.08.2022
(73) Patentinhaber: Henkel AG & Co. KGaA, 40589 Düsseldorf (DE)
(72) Erfinder: KRUCK, Constanze, 41515 Grevenbroich (DE); HILBIG, Sandra, 44894 Bochum (DE); MOCH, Melanie, 41542 Dormagen (DE); KESSLER-BECKER, Daniela, 51371 Leverkusen (DE)
(86) Internationale Anmeldenummer: PCT/EP2020/074202
(87) Internationale Veröffentlichungsnummer: WO 2021/058230

(56) Entgegenhaltungen:
- EP-A1- 3 501 486
- DE-A1- 102014 218 006
- DE-U1- 202005 002 951
- GB-A- 2 291 366

## Beschreibung

Gegenstand der vorliegenden Anmeldung ist ein Verfahren zum Färben von keratinischem Material, insbesondere menschlichen Haaren, welches die Anwendung von mindestens zwei verschiedenen Mitteln (a) und (b) umfasst. Das Mittel (a) stellt einen Premix bzw. ein Konzentrat dar, welcher mindestens ein aminofunktionalisiertes Silikonpolymer (a1) und mindestens eine farbgebende Verbindung (a2) enthält. Bei dem Mittel (b) handelt es sich um eine Träger-Formulierung, die mindestens eine Fettkomponente (b1) enthält. Die Mittel (a) und (b) sind durch einen reduzierten Wassergehalt gekennzeichnet. Vor der Anwendung wird durch Vermischen der Mittel (a) und (b) eine Anwendungsmischung hergestellt, welche auf das Keratinmaterial appliziert, einwirken gelassen und wieder abgewaschen wird.

Ein zweiter Gegenstand dieser Anmeldung ist eine Mehrkomponenten-Verpackungseinheit (Kit-of-parts) zum Färben von keratinischem Material, insbesondere menschlichen Haaren, welche getrennt voneinander konfektioniert in zwei verschiedenen Containern die Mittel (a) und (b) umfasst.

Die Veränderung von Form und Farbe von keratinischem Material, insbesondere von menschlichen Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

Der besondere Vorteil eines Haar-Maskara-Produktes liegt darin, dass die farbgebenden Verbindungen wie beispielsweise Pigmente nur in Form eines Films an der Oberfläche der Keratinfaser abgelagert werden. Die Beschaffenheit der Keratinfaser selbst wird somit bei der Anwendung des Produktes nicht verändert, so dass die Anwendung eines Haar-Maskara-Produktes mit einer besonders geringen Haarschädigung verbunden ist. Wünscht sich der Anwender seine Ursprungshaarfarbe zurück, so kann die Färbung schnell, vollständig und rückstandlos wieder von der Keratinfaser entfernt werden, ohne die Fasern zu schädigen oder die Ursprungshaarfarbe zu verändern. Die Entwicklung von auf Pigmenten basierenden Keratinfärbemitteln liegt daher voll im Trend.

Nach wie vor besteht bei diesem Färbesystem jedoch immer noch immer Optimierungsbedarf, wobei insbesondere die Farbintensität und das Haargefühl bzw. der Griff der keratinischen Fasern noch verbesserungswürdig sind.

Die Patentanmeldung DE 10 2014 218006 A1 betrifft ein Kit-of-parts, umfassend a) einen Container (C1) mit einem Mittel (M1), welches eine farbgebende Verbindung und ein bestimmtes aminiertes Silikonpolymer enthält, und b) einen Container (C2) mit einer Oxidationsmittelzubereitung (M2). Im Beispielteil offenbart diese Anmeldung ein Kit, worin die Mittel (E1) bzw. (E2) mit der Oxidationsmittelzubereitung (O1) im Gewichtsverhältnis 1:1 vermischt, auf Haarsträhnen aufgetragen und nach Einwirkung wieder ausgespült werden. Die Mittel (E1), (E2) und (O1) enthalten Wasser in einer Menge von 62,02 Gew.-%, 61,09 Gew.-% bzw.78,4 Gew.-%.

Es war die Aufgabe der vorliegenden Erfindung, ein Färbesystem bereitzustellen, das nach Möglichkeit mit der oxidativen Färbung vergleichbare Echtheitseigenschaften besitzt. Insbesondere die Waschechtheiten sollten herausragend sein, hierbei sollte jedoch auf den Einsatz der sonst zu diesem Zweck üblicherweise eingesetzten Oxidationsfarbstoffvorprodukte verzichtet werden. Es wurde nach einer Technologie gesucht, die es ermöglicht, die aus dem Stand der Technik bekannten farbgebenden Verbindungen (insbesondere Pigmente) in extrem dauerhafter Weise auf den Haaren zu fixieren. Bei Anwendung der Mittel in einem Färbeverfahren sollten intensive Färbeergebnisse mit guten Echtheitseigenschaften erzielt werden. Ein besonderer Fokus der Aufgabenstellung lag auf der Erzielung von intensiven Farbergebnissen bei gleichzeitig gutem Haargefühl.

Überraschenderweise hat sich nun herausgestellt, dass die vorgenannte Aufgabe hervorragend gelöst werden kann, wenn keratinische Materialien, insbesondere Haare, mit einem Verfahren gefärbt werden, bei welchem mindestens zwei Mittel (a) und (b) auf die keratinischen Materialien (Haare) appliziert werden. Hierbei enthält das Mittel (a) mindestens ein aminofunktionalisiertes Silikonpolymer (a1) und mindestens eine farbgebende Verbindung (a2). Das Mittel (a) liegt in Form eines Premixes bzw. eines Konzentrats vor, welches wasserarm bzw. wasserfrei konfektioniert ist und als Hauptbestanteile die Inhaltsstoffe (a1) und (a2) beinhaltet. Vor der Anwendung wird das Mittel (a) mit einer kosmetischen Trägerformulierung vermischt, die in Form des Mittels (b) vorliegt, die mindestens einen Fettbestandteil (b2) enthält und die sich durch einen reduzierten Wassergehalt (b1) auzeichnet. Die durch das Vermischen der Mittel (a) und (b) hergestellte Anwendungsmischung wird dann auf dem Keratinmaterial appliziert, einwirken gelassen und danach wieder mit Wasser ausgespült

Ein erster Gegenstand der vorliegenden Erfindung ist ein Verfahren zum Färben von keratinischem Material, insbesondere menschlichen Haaren, umfassend die folgenden Schritte:
(1) Bereitstellung eines Mittels (a), wobei das Mittel (a) - bezogen auf das Gesamtgewicht des Mittels (a) - 0 bis 10 Gew.-% Wasser und
   (a1) mindestens ein aminofunktionalisiertes Silikonpolymer, und
   (a2) mindestens eine farbgebende Verbindung enthält, und
(2) Bereitstellung eines Mittels (b), wobei das Mittel (b) - bezogen auf das Gesamtgewicht des Mittels (b) - enthält:
   (b1) 0 bis 50 Gew.-% Wasser und
   (b2) mindestens einen Fettbestandteil,
(3) Herstellung einer Anwendungsmischung durch Vermischen der Mittel (a) und (b),
(4) Applizieren der in Schritt (3) hergestellten Anwendungsmischung auf dem keratinischen Material,
(5) Einwirken der in Schritt (4) applizierten Anwendungsmischung auf dem keratinischen Material und
(6) Ausspülen der Anwendungsmischung mit Wasser.

Bei den zu dieser Erfindung führenden Arbeiten hat sich gezeigt, dass besonders intensive Farbergebnisse auf dem Keratinmaterial erhalten werden können, wenn das Mittel (a) in Form des zuvor beschriebenen Premixes bzw. Konzentrats bereitgestellt wird, wobei dieser Premix (a) erst kurz vor der Anwendung mit der Trägerformulierung (b) vermischt wird. Überraschenderweise lässt sich mit einer Anwendungsmischung, welche erst kurz vor der Anwendung durch Vermischen der beiden Mittel (a) und (b) erhalten wird, im Vergleich zu einer ansonsten gleichen Formulierung, welche alle Bestandteile der beiden Mittel (a) und (b) von Anfang an enthält, ein viel intensiveres Farbergebnis erhalten. Es konnte weiterhin gefunden werden, dass eine Reduktion des Wassergehalts im Mittel (b) zu einer Verbesserung des Griffgefühls auf den Keratinfasern führt.

### keratinisches Material

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

### Mittel (a)

In Schritt (1) des erfindungsgemäßen Verfahren wird das Mittel (a) bereitgestellt. Beispielsweise kann das Mittel (a) in einer Verpackungseinheit oder einem Container vorliegen und auf diese Weise dem Anwender zur Verfügung gestellt werden. Bei dem Container kann es sich beispielsweise um ein Sachet, eine Flasche, eine Dose, einen Tiegel oder auch ein anderes für kosmetische Formulierungen geeignetes Behältnis handeln.

Das Mittel (a) ist gekennzeichnet durch seinen Gehalt an den erfindungswesentlichen Bestandteilen (a), (a1) und (a2).

### aminofunktionalisiertes Silikonpolymer (a1) im Mittel (a)

Als ersten erfindungswesentlichen Inhaltsstoff (a1) enthält das Mittel (a) mindestens ein aminofunktionalisiertes Silikonpolymer. Das aminofunktionalisiertes Silikonpolymer kann alternativ auch als Aminosilikon oder Amodimethicone bezeichnet werden.

Silikonpolymere sind im allgemeinen Makromoleküle mit einem Molekulargewicht von mindestens 500 g/mol, bevorzugt mindestens 1000 g/mol, weiter bevorzugt von mindestens 2500 g/mol, besonders bevorzugt von mindestens 5000 g/mol, welche sich wiederholende organische Einheiten umfassen.

Das maximale Molekulargewicht des Silikonpolymers hängt von dem Polymerisationsgrad (Anzahl der polymerisierten Monomere) und der Ansatzgröße ab und wird durch die Polymerisationsmethode mitbestimmt. Im Sinne der vorliegenden Erfindung ist es bevorzugt, wenn das maximale Molekulargewicht des Silikonpolymers nicht mehr als 10⁷ g/mol, bevorzugt nicht mehr als 10⁶ g/mol und besonders bevorzugt nicht mehr als 10⁵ g/mol beträgt.

Die Silikonpolymere umfassen viele Si-O-Wiederholungseinheiten, wobei die Si-Atome organische Reste wie beispielsweise Alkylgruppen oder substituierte Alkylgruppen tragen können. Alternativ wird ein Silikonpolymer daher auch als Polydimethylsiloxan bezeichnet.

In Entsprechung des hohen Molekulargewichts der Silikonpolymere basieren diese auf mehr als 10 Si-O Wiederholungseinheiten, bevorzugt mehr als 50 Si-O-Wiederholungseinheiten und besonders bevorzugt mehr als 100 Si-O-Wiederholungseinheiten, ganz besonders bevorzugt mehr als 500 Si-O-Wiederholungseinheiten.

Unter einem aminofunktionalisierten Silikonpolymer wird ein funktionalisiertes Silikon verstanden, welches mindestens eine Struktureinheit mit einer Aminogruppe trägt. Bevorzugt trägt das aminofunktionalisierte Silikonpolymer mehrere Struktureinheiten mit jeweils mindestens einer Aminogruppe. Unter einer Aminogruppe wird eine primäre Aminogruppe, eine sekundäre Aminogruppe und eine tertiäre Aminogruppe verstanden. Alle diese Aminogruppen können im sauren Milieu protoniert werden und liegen dann in ihrer kationischen Form vor.

Prinzipiell konnten gute Effekte aminofunktionalisierten Silikonpolymeren (a1) erzielt werden, wenn diese mindestens eine primäre, mindestens eine sekundäre und/oder mindestens eine tertiäre Aminogruppe tragen. Färbungen mit der besten Waschechtheit wurden jedoch beobachtet, wenn ein aminofunktionalisiertes Silikonpolymer (a1) im Mittel (a) eingesetzt wurde, welches mindestens eine sekundäre Aminogruppe enthält.

In einer ganz besonders bevorzugten Ausführungsform ist ein erfindungsgemäßes Verfahren dadurch gekennzeichnet, dass das Mittel (a) mindestens ein aminofunktionalisiertes Silikonpolymer (a1) mit mindestens eine sekundären Aminogruppe enthält.

Die sekundäre Aminogruppe(n) kann bzw. können sich an verschiedenen Positionen des aminofunktionalisierten Silikonpolymers befinden. Ganz besonders gute Effekte wurden gefunden, wenn ein aminofunktionalisiertes Silikonpolymer (a1) eingesetzt wurde, dass mindestens eine, bevorzugt mehrere Struktureinheiten der Formel (Si-Amino) besitzt.

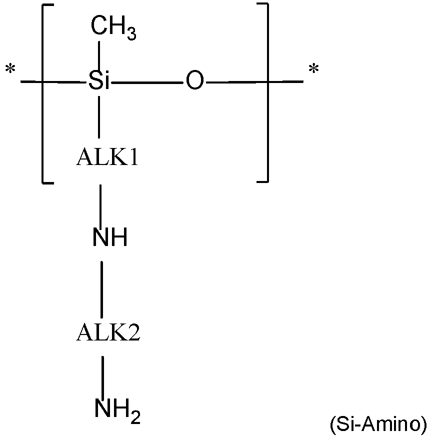

In den Struktureinheiten der Formel (Si-Amino) steht die Kürzel ALK1 und ALK2 unabhängig voneinander für eine lineare oder verzweigte, zweiwertige C₁-C₂₀-Alkylengruppe.

In einer weiteren ganz besonders bevorzugten Ausführungsform ist ein erfindungsgemäßes Verfahren dadurch gekennzeichnet, dass das Mittel (a) mindestens ein aminofunktionalisiertes Silikonpolymer (a1) enthält, das mindestens eine Struktureinheit der Formel (Si-Amino) umfasst, 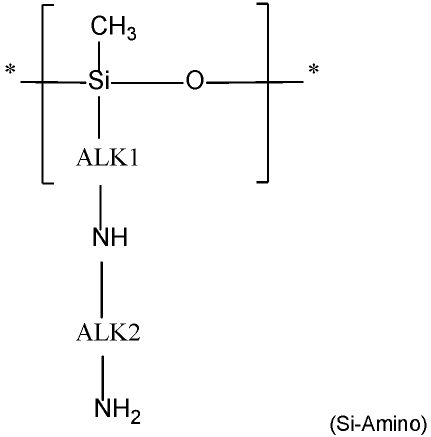 wobei
- ALK1 und ALK2: unabhängig voneinander für eine lineare oder verzweigte, zweiwertige C₁-C₂₀-Alkylengruppe stehen.

Die mit einem Stern (*) gekennzeichneten Position geben hierbei jeweils die Bindung zu weiteren Struktureinheiten des Silikonpolymers an. Beispielsweise kann das dem Stern benachbarte Silicium-Atom an ein weiteres Sauerstoffatom gebunden sein, und das dem Stern benachbarte Sauerstoffatom kann an ein weiteres Siliciumatom oder auch an eine C₁-C₆-Alkylgruppe gebunden sein.

Eine zweiwertige C₁-C₂₀-Alkylengruppe kann alternativ auch als eine divalente oder zweibindige C₁-C₂₀-Alkylengruppe bezeichnet werden, womit gemeint ist, dass jede Gruppierung ALK1 bzw. AK2 zwei Bindungen eingehen kann.

Im Fall von ALK1 erfolgt eine Bindung vom Silicium-Atom zur Gruppierung ALK1, und die zweite Bindung besteht zwischen ALK1 und der sekundären Aminogruppe.

Im Fall von ALK2 erfolgt eine Bindung von der sekundären Aminogruppe zur Gruppierung ALK2, und die zweite Bindung besteht zwischen ALK2 und der primären Aminogruppe.

Beispiele für eine lineare zweiwertige C₁-C₂₀-Alkylengruppe sind beispielsweise die Methylen-gruppe (-CH₂-), die Ethylengruppe (-CH₂-CH₂-), die Propylengruppe (-CH₂-CH₂-CH₂-) und die Butylengruppe (-CH₂-CH₂-CH₂-CH₂-). Die Propylengruppe (-CH₂-CH₂-CH₂-) ist besonders bevorzugt. Ab einer Kettenlänge von 3 C-Atomen können zweiwertige Alkylengruppen auch verzweigt sein. Beispiele für verzweigte, zweiwertige C₃-C₂₀-Alkylengruppen sind (-CH₂-CH(CH₃)-) und (-CH₂-CH(CH₃)-CH₂-).

In einer weiteren besonders bevorzugten Ausführungsform stellen die Struktureinheiten der Formel (Si-Amino) Wiederholungseinheiten im aminofunktionalisierten Silikonpolymer (a1) dar, so dass das Silikonpolymer mehrer Struktureinheiten der Formel (Si-Amino) umfasst.

Im Folgenden werden besonders gut geeignete aminofunktionalisierte Silikonpolymere (a1) mit mindestens einer sekundären Aminogruppe aufgelistet.

Färbungen mit den allerbesten Waschechtheiten konnten erhalten werden, wenn im erfindungsgemäßen Verfahren mindestens ein Mittel (a) auf dem keratinischen Material appliziert wurde, das mindestens ein aminofunktionalisiertes Silikonpolymer (a1) enthält, das Struktureinheiten der Formel (Si-I) und der Formel (Si-II) umfasst

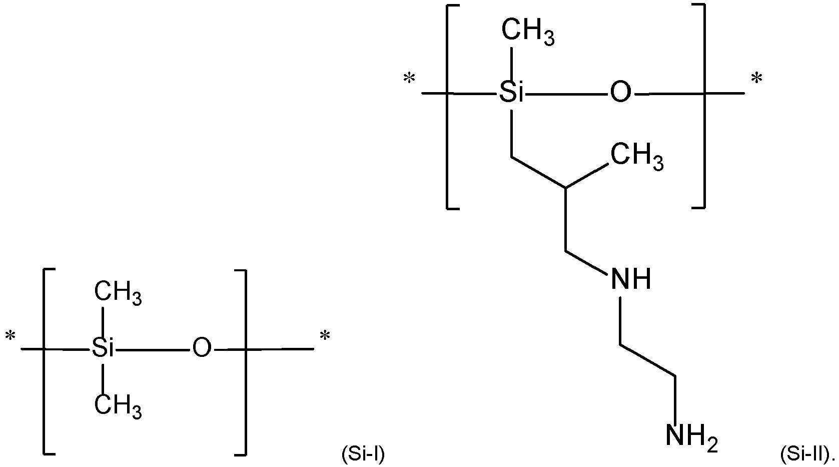

In einer weiteren explizit ganz besonders bevorzugten Ausführungsform ist ein erfindungsgemäßes Verfahren dadurch gekennzeichnet, dass das Mittel (a) mindestens ein aminofunktionalisiertes Silikonpolymer (a1) enthält, das Struktureinheiten der Formel (Si-I) und der Formel (Si-II) umfasst

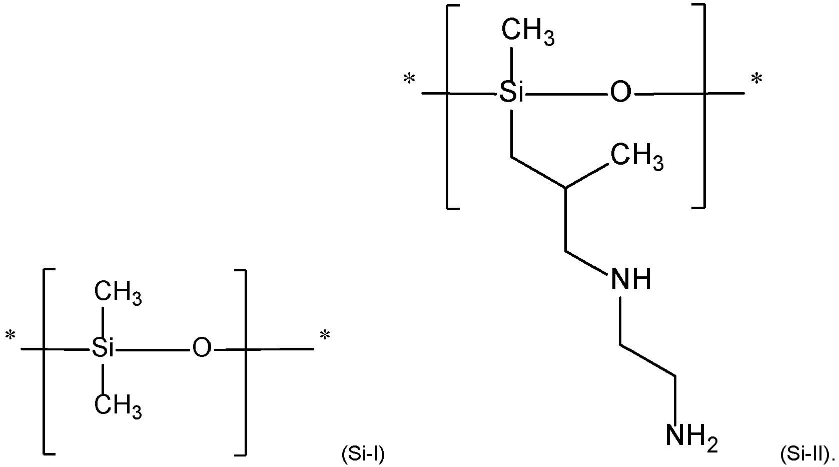

Ein entsprechendes aminofunkionalisiertes Silikonpolymer mit den Struktureinheiten (Si-I) und (Si-II) ist beispielweise das Handelsprodukt DC 2-8566 bzw. Dowsil 2-8566 Amino Fluid, das von der Firma Dow Chemical Company komerziell vertrieben wird und welches die Benennung "Siloxanes and Silicones, 3-[(2-Aminoethyl)amino]-2-methylpropyl Me, Di-Me-Siloxane" sowie die CAS-Nummer 106842-44-8 trägt.

Im Rahmen einer weiteren bevorzugten Ausführungsform ist ein erfindungsgemäßes Verfahren gekennzeichnet durch die Anwendung eines Mittels (a) auf dem keratinischem Material, wobei das Mittel (a) mindestens ein aminofunktionelles Silikonpolymer (a1) der Formel der Formel (Si-III) enthält, 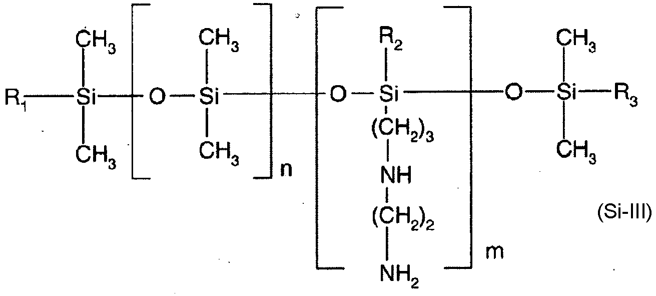 wobei
- m und n bedeuten Zahlen, die so gewählt sind, daß die Summe (n + m) im Bereich von 1 bis 1000 liegt,
- n ist eine Zahl im Bereich von 0 bis 999 und m ist eine Zahl im Bereich von 1 bis 1000,
- R1, R2 und R3, die gleich oder verschieden sind, bedeuten eine Hydroxygruppe oder eine C1-4-Alkoxygruppe,
- wobei mindestens eine der Gruppen R1 bis R3 eine Hydroxygruppe bedeutet;

Weitere erfindungsgemäß bevorzugte Verfahren sind gekennzeichnet durch die Anwendung eines Mittels (a) auf dem keratinischem Material, wobei das Mittel (a) mindestens aminofunktionelles Silikonpolymer (a1) der Formel der Formel (Si-IV) enthält, 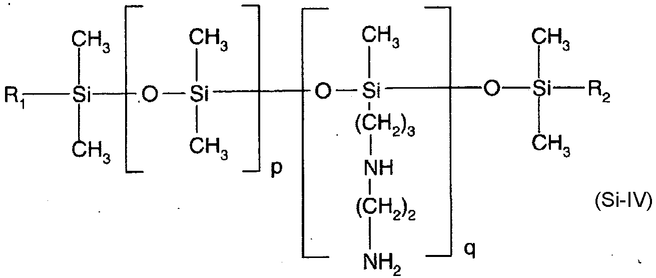 in der
- p und q bedeuten Zahlen, die so gewählt sind, daß die Summe (p + q) im Bereich von 1 bis 1000 liegt,
- p ist eine Zahl im Bereich von 0 bis 999 und q ist eine Zahl im Bereich von 1 bis 1000,
- R1 und R2, die verschieden sind, bedeuten eine Hydroxygruppe oder eine C1-4-Alkoxygruppe, wobei mindestens eine der Gruppen R1 bis R2 eine Hydroxygruppe bedeutet.

Die Silikone der Formeln (Si-III) und (Si-IV) unterscheiden sich durch die Gruppierung am Si-Atom, das die stickstoffhaltige Gruppe trägt: In Formel (Si-III) bedeutet R2 eine Hydroxygruppe oder eine C1-4-Alkoxygruppe, während der Rest in Formel (Si-IV) eine Methylgruppe ist. Die einzelnen Si-Gruppierungen, die mit den Indices m und n bzw. p und q gekennzeichnet sind, müssen nicht als Blöcke vorliegen, vielmehr können die einzelnen Einheiten auch statistisch verteilt vorliegen, d.h. in den Formeln (Si-III) und (Si-IV) ist nicht zwingend jedes R1-Si(CH₃)₂-Gruppe an eine -[O-Si(CH₃)₂]-Gruppierung gebunden.

Als besonders wirkungsvoll im Hinblick auf die gewünschten Effekte haben sich auch erfindungsgemäßen Verfahren erwiesen, in welchen ein Mittel (a) auf den Keratinfasern appliziert wird, welches mindestens ein aminofunktionelles Silikonpolymer (a1) der Formel der Formel (Si-V) enthält 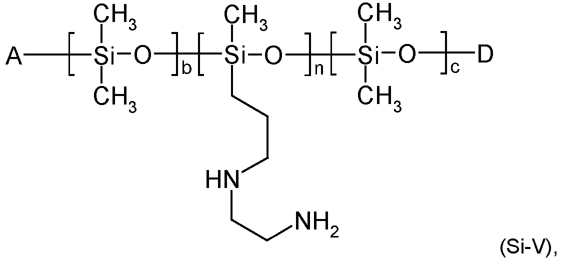 in der
- A: für eine Gruppe -OH, -O-Si(CH₃)₃,-O-Si(CH₃)₂OH ,-O-Si(CH₃)₂OCH₃ steht,
- D: für eine Gruppe -H, -Si(CH₃)₃,-Si(CH₃)₂OH, -Si(CH₃)₂OCH₃ steht,
- b, n und c: für ganze Zahlen zwischen 0 und 1000 stehen,
mit den Maßgaben
- n > 0 und b + c > 0
- mindestens eine der Bedingungen A = -OH bzw. D = -H ist erfüllt.

In der vorstehend genannten Formel (Si-V) sind die einzelnen Siloxaneinheiten mit den Indices b, c und n statistisch verteilt, d.h. es muß sich nicht zwingend um Blockcopolymere handeln.

Das Mittel (a) kann weiterhin auch ein oder mehrere verschiedene aminofunktionalisierte Silikonpolymere enthalten, die durch die Formel (Si-VI)

M(RₐQ_{b}SiO_{(4-a-b)/2)x}(R_{c}SiO_{(4-c)/2)y}M (Si-VI)

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹HZ ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stand der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, - CH₂CH(CH₃)CH₂-, Phenylen, Naphthylen, -CH₂CH₂SCH₂CH ₂-, -CH₂CH₂OCH₂-, -OCH₂CH₂-, -OCH₂ CH₂CH₂-, -CH₂CH(CH₃)C(O)OCH₂-, -(CH₂)₃ CC(O)OCH₂CH₂-, -C₆H ₄C₆H₄-, -C₆H ₄CH₂C₆H₄-; und -(CH ₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂ )_{z}NH₂, worin z 1 oder mehr ist. Eine andere mögliche Formel für Z ist -NH(CH₂)_{z}(CH ₂)_{zz}NH, worin sowohl z als auch zz unabhängig 1 oder mehr sind, wobei diese Struktur Diamino-Ringstrukturen umfaßt, wie Piperazinyl. Z ist am bevorzugtesten ein -NHCH₂CH ₂NH₂-Rest. Eine andere mögliche Formel für Z ist - N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel -CH₂CH₂CH₂NHCH₂CH₂NH ₂. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der RₐQ_{b} SiO_{(4-a-b)/2}-Einheiten zu den R_{c}SiO_{(4-c)/2}-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Im Rahmen einer besonders bevorzugten Ausführungsform ist ein erfindungsgemäßes Verfahren gekennzeichnet durch die Anwendung eines Mittels (a) auf dem keratinischem Material, wobei das Mittel (a) ein aminofunktionelles Silikonpolymer der Formel (Si-VII)

R'ₐG₃₋ₐ-Si(OSiG ₂)ₙ-(OSiG _{b}R'_{2- b})ₘ-O-SiG₃₋ₐ-R'ₐ (Si-VII),

enthält, worin bedeutet:
- G ist-H, eine Phenylgruppe, -OH, -O-CH₃, -CH₃, -O-CH₂CH₃, -CH₂CH₃, -O-CH₂CH₂CH₃,-CH₂CH₂CH₃, -O-CH(CH₃)₂, -CH(CH₃)₂, -O-CH₂CH₂CH₂CH₃, - CH₂CH₂CH₂CH₃, -O-CH₂CH(CH₃)₂, -CH₂CH(CH₃)₂, -O-CH(CH₃)CH₂CH₃, - CH(CH₃)CH₂CH₃, -O-C(CH₃)₃, -C(CH₃)₃ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   ∘ -Q-N(R")-CH₂-CH₂-N(R")₂
   ∘ -Q-N(R")₂
   ∘ -Q-N⁺(R")₃A⁻
   ∘ -Q-N⁺H(R")₂ A⁻
   ∘ -Q-N⁺H₂(R")A⁻
   ∘ -Q-N(R")-CH₂-CH₂-N⁺R"H₂A⁻,

wobei jedes Q für eine chemische Bindung, -CH₂-, -CH₂-CH₂-, -CH₂CH₂CH₂-, -C(CH₃)₂-, -CH₂CH₂CH₂CH₂-, -CH₂C(CH₃)₂-, -CH(CH₃)CH₂CH₂- steht,
R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, - CH(CH₃)₂, -CH₂CH₂CH₂H₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

Im Rahmen einer weiteren bevorzugten Ausführungsform ist ein erfindungsgemäßes Verfahren gekennzeichnet durch die Anwendung eines Mittels (a) auf dem keratinischem Material, wobei das Mittel (a) mindestens ein aminofunktionelles Silikonpolymer (a1) der Formel (Si-Vlla) enthält, 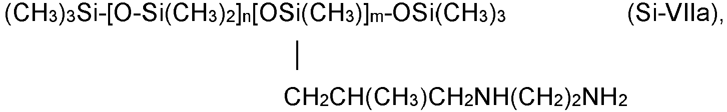 worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet.

Im Rahmen einer weiteren bevorzugten Ausführungsform ist ein erfindungsgemäßes Verfahren gekennzeichnet durch die Anwendung eines Mittels (a) auf dem keratinischem Material, wobei das Mittel (a) mindestens ein aminofunktionelles Silikonpolymer der Formel (Si-Vllb) enthält 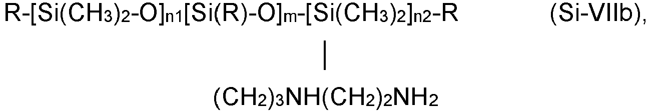 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese aminofunktionalisierten Siliconpolymere werden nach der INCI-Deklaration als Amodimethicone bezeichnet.

Unabhängig davon, welche aminofunktionellen Silicone eingesetzt werden, sind erfindungsgemäße Mittel (a) bevorzugt, die ein aminofunktionelles Silikonpolymer enthalten, dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Weiterhin sind auch Mittel (a) zum Einsatz im erfindungsgemäßen Verfahren geeignet, welche ein spezielles 4-Morpholinomethyl-substituiertes Silikonpolymer (a1) enthielten. Dieses aminofunktionalisierte Silikonpolymer umfasst Struktureinheiten der Formeln (SI-VIII) und der Formel (Si-IX)

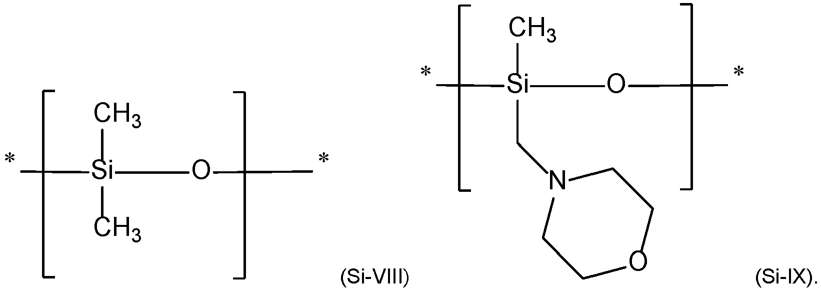

Entsprechende 4-Morpholinomethyl-substituiertes Silikonpolymere werden im folgenden beschrieben.

Ein ganz besonders bevorzugtes aminofunktionaliserte Silikonpolymer ist unter dem Namen Amodimethicone/Morpholinomethyl Silsesquioxane Copolymer bekannt und in Form des Rohstoffes Belsil ADM 8301 E von Wacker kommerziell erhältlich.

Als 4-morpholinomethyl-substituiertes Silikon kann beispielsweise ein Silikon eingesetzt werden, welches Struktureinheiten der Formeln (Si-VIII), (Si-IX) und (Si-X) aufweist 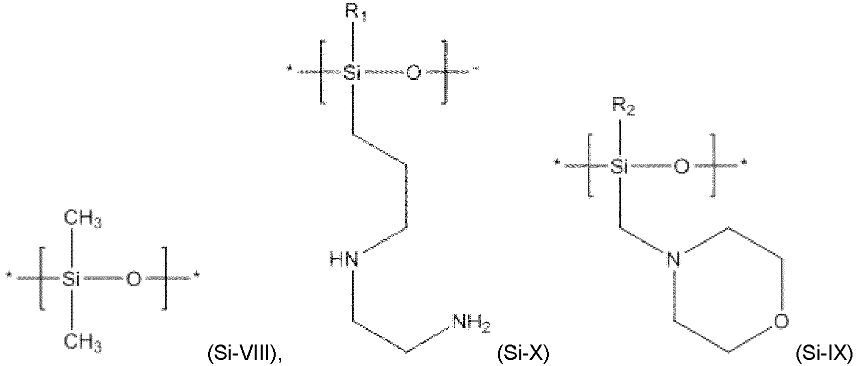 in denen
- R1: für -CH₃, -OH, -OCH₃, -O-CH₂CH₃, -O-CH₂CH₂CH₃, oder -O-CH(CH₃)₂ steht;
- R2: für -CH₃, -OH, oder -OCH₃ steht.

Besonders bevorzugte erfindungsgemäße Mittel (a) enthalten mindestens ein 4-morpholinomethylsubstituierten Silikons der Formel (Si-XI) 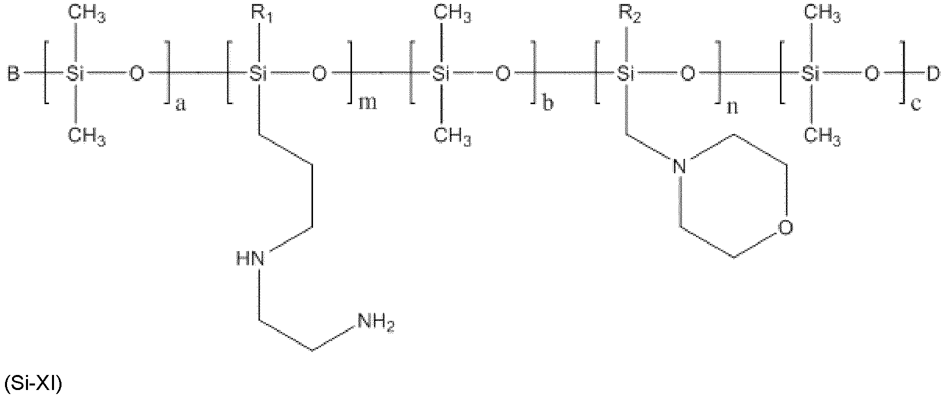 in der
- R1: für -CH₃, -OH, -OCH₃, -O-CH₂CH₃, -O-CH₂CH₂CH₃, oder -O-CH(CH₃)₂ steht;
- R2: für -CH₃, -OH, oder -OCH₃ steht.
- B: für eine Gruppe -OH, -O-Si(CH₃)₃,-O-Si(CH₃)₂OH ,-O-Si(CH₃)₂OCH₃ steht,
- D: für eine Gruppe -H, -Si(CH₃)₃,-Si(CH₃)₂OH, -Si(CH₃)₂OCH₃ steht,

a, b und c unabhängig voneinander für ganze Zahlen zwischen 0 und 1000 stehen, mit der Maßgabe a + b + c > 0
m und n unabhängig voneinander für ganze, Zahlen zwischen 1 und 1000 stehen mit den Maßgabe, daß
   - mindestens eine der Bedingungen B = -OH bzw. D = -H erfüllt ist,
   - die Einheiten a, b, c, m und n statistisch oder blockweise im Molekül verteilt vorliegen.

Strukturformel (Si-XI) soll verdeutlichen, daß die Siloxangruppen n und m nicht zwingend direkt an eine Endgruppierung B bzw. D gebunden sein müssen. Vielmehr gilt in bevorzugten Formeln (Si-VI) a > 0 oder b > 0 und in besonders bevorzugten Formeln (Si-VI) a > 0 und c > 0, d.h. die terminale Gruppierung B bzw. D ist vorzugsweise an eine Dimethylsiloxy-Gruppierung gebunden. Auch in Formel (Si-VI) sind die Siloxaneinheiten a, b, c, m und n vorzugsweise statistisch verteilt.
Die durch Formel (Si-VI) dargestellten erfindungsgemäß eingesetzten Silikone können trimethylsilylterminiert sein (D oder B = -Si(CH₃)₃), sie können aber auch zweiseitig dimethylsilylhydroxy- oder einseitig dimethylsilylhydroxy- und dimethylsilylmethoxy-terminiert sein. Im Rahmen der vorliegenden Erfindung besonders bevorzugt eingesetzte Silikone sind ausgewählt aus Silikonen, in denen

| | |
|---|---|
| B = -O-Si(CH₃)₂OH und | D = -Si(CH₃)₃ |
| B = -O-Si(CH₃)₂OH und | D = -Si(CH₃)₂OH |
| B = -O-Si(CH₃)₂OH und | D = -Si(CH₃)₂OCH₃ |
| B = -O-Si(CH₃)₃ | und D = -Si(CH₃)₂OH |
| B = -O-Si(CH₃)₂OCH₃ | und D = -Si(CH₃)₂OH |

bedeutet. Diese Silikone führen zu exorbitanten Verbesserungen der Haareigenschaften der mit den erfindungsgemäßen Mitteln behandelten Haare, und zu einem gravierend verbesserten Schutz bei oxidativer Behandlung.

Bei dem im erfindungsgemäßen Verfahren eingesetzten Mittel (a) handelt es sich um einen Premix bzw. um ein Konzentrat, welcher die aminofunkionaliserten Silikonpolymere (a1) als einen Hauptbestandteil enthält.

Es ist bevorzugt, wenn das Mittel (a) alle Hauptbestandteile in entpsprechend hohen Mengen enthält. Besonders gute Ergebnisse konnten erhalten werden, wenn das Mittel (a) - bezogen auf das Gesamtgewicht des Mittels (a) - ein oder mehrere aminofunktionalisierte Silikonpolymere (a1) in einer Gesamtmenge von 2,0 bis 95,0 Gew.-%, bevorzugt 4,0 bis 70,0 Gew.-%, weiter bevorzugt von 6,0 bis 50,0 Gew.-% und ganz besonders bevorzugt von 8,0 bis 20,0 Gew.-% enthält.

Im Rahmen einer weiteren besonders bevorzugten Ausführungsform ist ein erfindungsgemäßes Verfahren dadurch gekennzeichnet, dass das Mittel (a) - bezogen auf das Gesamtgewicht des Mittels (a) - ein oder mehrere aminofunktionalisierte Silikonpolymere (a1) in einer Gesamtmenge von 2,0 bis 95,0 Gew.-%, bevorzugt 4,0 bis 70,0 Gew.-%, weiter bevorzugt von 6,0 bis 50,0 Gew.-% und ganz besonders bevorzugt von 8,0 bis 20,0 Gew.-% enthält.

### farbgebende Verbindung (a2) im Mittel (a)

Als zweiten erfindungswesentlichen Bestandteil enthält das im erfindungsgemäßen Verfahren eingesetzte Mittel (a) mindestens eine farbgebende Verbindung (a2).

Unter farbgebenden Verbindungen werden im Sinne der Erfindung Substanzen verstanden, die in der Lage sind, dem Keratinmaterial eine Färbung zu verleihen. Besonders gut geeignete farbgebende Verbindungen können ausgewählt werden aus der Gruppe der Pigmente, der direktziehenden Farbstoffe, der photochromen Farbstoffe und der thermochromen Farbstoffe.

Im Rahmen einer weiteren bevorzugten Ausführungsform ist ein erfindungsgemäßes Verfahren dadurch gekennzeichnet, dass das Mittel (a) mindestens eine farbgebende Verbindung (a2) aus der Gruppe der Pigmente, der direktziehenden Farbstoffe, der photochromen Farbstoffe und der thermochromen Farbstoffe enthält.

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Farbpigmente können anorganischen und/oder organischen Ursprungs sein.

In einer bevorzugten Ausführungsform ist ein erfindungsgemäßes Mittel (a) dadurch gekennzeichnet, dass es mindestens eine farbgebende Verbindung (a2) aus der Gruppe der anorganischen und/oder organischen Pigmente enthält.

Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß ebenfalls besonders bevorzugte Farbpigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

In einer weiteren bevorzugten Ausführungsform ist ein erfindungsgemäßes Verfahren dadurch gekennzeichnet, dass das Mittel (a) mindestens eine farbgebende Verbindung (a2) aus der Gruppe der anorganischen Pigmente enthält, das bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

In einer weiteren bevorzugten Ausführungsform ist ein erfindungsgemäßes Mittel dadurch gekennzeichnet, dass es (a) mindestens eine farbgebende Verbindung (a2) aus der Gruppe der Pigmente enthält, die ausgewählt ist aus Pigmenten auf Mica- oder Glimmerbasis, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Beispiele für besonders geeignete Farbpigmente sind im Handel beispielsweise unter den Handelsbezeichnungen Rona^{®}, Colorona^{®}, Xirona^{®}, Dichrona^{®} und Timiron^{®} von der Firma Merck, Ariabel^{®} und Unipure^{®} von der Firma Sensient, Prestige^{®} von der Firma Eckart Cosmetic Colors und Sunshine^{®} von der Firma Sunstar erhältlich.

Ganz besonders bevorzugte Farbpigmente mit der Handelsbezeichnung Colorona^{®} sind beispielsweise:
Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491 (Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491(Iron oxides)
Colorona Blackstar Gold, Merck, MICA, CI 77499 (IRON OXIDES)

Weiterhin besonders bevorzugte Farbpigmente mit der Handelsbezeichnung Xirona^{®} sind beispielsweise:
Xirona Golden Sky, Merck, Silica, CI 77891 (Titanium Dioxide), Tin Oxide
Xirona Caribbean Blue, Merck, Mica, CI 77891 (Titanium Dioxide), Silica, Tin Oxide
Xirona Kiwi Rose, Merck, Silica, CI 77891 (Titanium Dioxide), Tin Oxide
Xirona Magic Mauve, Merck, Silica, CI 77891 (Titanium Dioxide), Tin Oxide.

Zudem sind besonders bevorzugte Farbpigmente mit der Handelsbezeichnung Unipure^{®} beispielsweise:
Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Im Rahmen einer weiteren Ausführungsform kann das erfindungsgemäße Mittel auch (a) ein oder mehrere farbgebende Verbindungen (a2) aus der Gruppe der organischen Pigmente enthalten

Bei den erfindungsgemäßen organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470 genannt werden.

In einer weiteren besonders bevorzugten Ausführungsform ist ein erfindungsgemäßes Verfahren dadurch gekennzeichnet, dass das Mittel (a) mindestens eine farbgebende Verbindungen (a2) aus der Gruppe der organischen Pigmente enthält, die bevorzugt ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Aufgrund ihrer ausgezeichneten Licht- und Temperaturbeständigkeit ist die Verwendung der zuvor genannten Pigmente in dem Mittel (a) des erfindungsgemäßen Verfahrens ganz besonders bevorzugt. Weiterhin ist es bevorzugt, wenn die eingesetzten Pigmente eine bestimmte Teilchengröße aufweisen. Es ist daher erfindungsgemäß vorteilhaft, wenn das mindestens eine Pigment eine mittlere Teilchengröße D₅₀ von 1,0 bis 50 µm, vorzugsweise von 5,0 bis 45 µm, bevorzugt von 10 bis 40 µm, insbesondere von 14 bis 30 µm, aufweist. Die mittlere Teilchengröße D₅₀ kann beispielsweise unter Verwendung von dynamischer Lichtstreuung (DLS) bestimmt werden.

Die farbgebenden Verbindungen (a2), inbesondere die farbgebenden Verbindungen aus der Gruppe der Pigmente, stellen den zweiten Hauptbestandteil des erfindungsgemäßen Mittels (a) bzw. des Premixes dar. Als weitere Hauptkomponente (a2) werden auch das oder die Pigmente ganz besonders bevorzugt in entsprechend höheren Mengen im Mittel (a) eingsetzt. Besonders gute Ergebnisse wurden erhalten, wenn das Mittel (a)- bezogen auf das Gesamtgewicht des Mittels (a) - ein odere mehrere Pigmente in einer Gesamtmenge von 2,0 bis 95,0 Gew.-%, bevorzugt 4,0 bis 70,0 Gew.-%, weiter bevorzugt von 6,0 bis 50,0 Gew.-% und ganz besonders bevorzugt von 8,0 bis 30,0 Gew.-% enthielt.

In einer weiteren ganz besonders bevorzugten Ausführungsform ist ein erfindungsgemäßes Mittel dadurch gekennzeichnet, dass das Mittel (a) - bezogen auf das Gesamtgewicht des Mittels (a) - ein odere mehrere Pigmente in einer Gesamtmenge von 2,0 bis 95,0 Gew.-%, bevorzugt 4,0 bis 70,0 Gew.-%, weiter bevorzugt von 6,0 bis 50,0 Gew.-% und ganz besonders bevorzugt von 8,0 bis 30,0 Gew.-% enthält.

Als farbgebende Verbindungen (a2) können die im erfindungsgemäßen Verfahren angewendeten Mittel (a) auch einen oder mehrere direktziehende Farbstoffe enthalten. Bei direktziehenden Farbstoffen handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone, Triarylmethanfarbstoffe oder Indophenole.

Die direktziehenden Farbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die direktziehenden Farbstoffe im Sinne der vorliegenden Erfindung eine Löslichkeit in Wasser (760 mmHg) bei 25 °C von mehr als 1,0 g/L.

Direktziehende Farbstoffe können in anionische, kationische und nichtionische direktziehende Farbstoffe unterteilt werden.

In einer weiteren Ausführungsform ist ein erfindungsgemäßes Verfahren dadurch gekennzeichnet, dass das Mittel (a) mindestens eine farbgebende Verbindung (a2) aus der Gruppe der anionischen, nichtionischen und kationischen direktziehenden Farbstoffe enthält.

Geeignete kationische direktziehende Farbstoffe sind beispielsweise Basic Blue 7, Basic Blue 26, HC Blue 16, Basic Violet 2 und Basic Violet 14, Basic Yellow 57, Basic Red 76, Basic Blue 16, Basic Blue 347 (Cationic Blue 347 / Dystar), HC Blue No. 16, Basic Blue 99, Basic Brown 16, Basic Brown 17, Basic Yellow 57, Basic Yellow 87, Basic Orange 31, Basic Red 51 Basic Red 76.

Als nichtionische direktziehende Farbstoffe können beipsielsweise nichtionische Nitro- und Chinonfarbstoffe und neutrale Azofarbstoffe eingesetzt werden. Geeigente nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Im Zuge der zu dieser Erfindung führenden Arbeiten hat sich herausgestellt, dass auch mit Mitteln (a) die mindestens einen anionischen direktziehenden Farbstoff (a2) enthalten, Färbungen mit guten Farbintensitäten und Echtheiten erzeugt werden können.

In einer weiteren Ausführungsform ist ein erfindungsgemäßes Verfahren daher dadurch gekennzeichnet, dass das Mittel (a) mindestens einen anionischen direktziehenden Farbstoff enthält.

Anionische direktziehende Farbstoffe werden auch als Säurefarbstoffe bezeichnet. Unter Säurefarbstoffen werden direktziehende Farbstoffe verstanden, die mindestens eine Carbonsäuregruppierung (-COOH) und/oder eine Sulfonsäuregruppierung (-SO₃H) besitzen. In Abhängigkeit vom pH-Wert liegen die protonierten Formen (-COOH, -SO₃H) der Carbonsäure- bzw. Sulfonsäuregruppierungen mit ihren deprotonierten Formen (-COO⁻, -SO₃⁻ vor) im Gleichgewicht vor. Mit abnehmendem pH-Wert steigt der Anteil der protonierten Formen. Werden direktziehende Farbstoffe in Form ihrer Salze eingesetzt, so liegen die Carbonsäuregruppen bzw. Sulfonsäuregruppen in deprotonierter Form vor und sind zur Einhaltung der Elektroneutralität mit entsprechenden stöchiometrischen Äquivalente an Kationen neutralisiert. Erfindungsgemäße Säurefarbstoffe können auch in Form ihrer Natriumsalze und/oder ihrer Kaliumsalze eingesetzt werden.

Die Säurefarbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die Säurefarbstoffe im Sinne der vorliegenden Erfindung besitzen eine Löslichkeit in Wasser (760 mmHg) bei 25 °C von mehr als 1,0 g/L.

Die Erdalkalisalze (wie beispielsweise Calciumsalze und Magnesiumsalze) bzw. Aluminiumsalze von Säurefarbstoffen besitzen oftmals eine schlechtere Löslichkeit als die entsprechenden Alkalisalze. Sofern die Löslichkeit dieser Salze unterhalb von 0,5 g/L (25 °C, 760 mmHg) liegt, fallen diese nicht unter die Definition eines direktziehenden Farbstoffes.

Ein wesentliches Merkmal der Säurefarbstoffe ist ihr Vermögen, anionische Ladungen auszubilden, wobei die hierfür verantwortlichen Carbonsäure- bzw. Sulfonsäuregruppen üblicherweise an verschiedene chromophore Systeme geknüpft sind. Geeignete chromophore Systeme finden sich beispielsweise in den Strukturen von Nitrophenylendiaminen, Nitroaminophenolen, Azofarbstoffen, Anthrachinonfarbstoffen, Triarylmethanfarbstoffen, Xanthen-Farbstoffen, Rhodamin-Farbstoffen, Oxazinfarbstoffen und/oder Indophenolfarbstoffen.

Im Rahmen einer weiteren Ausführungsform ist ein Verfahren zum Färben von keratinischem Material dadurch gekennzeichnet ist, dass das Mittel (a) mindestens einen anionischen direktziehenden Farbstoff enthält, der ausgewählt ist aus der Gruppe der Nitrophenylendiamine, der Nitroaminophenole, der Azofarbstoffe, der Anthrachinonfarbstoffe, der Triarylmethanfarbstoffe, der Xanthen-Farbstoffe, der Rhodamin-Farbstoffe, der Oxazinfarbstoffen und/oder der Indophenolfarbstoffe, wobei die Farbstoffe aus der vorgenannten Gruppe jeweils mindestens eine Carbonsäuregruppe (-COOH), eine Natriumcarboxylatgruppe (-COONa), eine Kaliumcarboxylatgruppe (-COOK), eine Sulfonsäuregruppe (-SO₃H) eine Natriumsulfonatgruppe (-SO₃Na) und/oder eine Kaliumsulfonatgruppe (-SO₃K) besitzen.

Als geeignete Säurefarbstoffe können beispielsweise eine oder mehrere Verbindungen aus der folgenden Gruppe ausgewählt werden: Acid Yellow 1 (D&C Yellow 7, Citronin A, Ext. D&C Yellow No. 7, Japan Yellow 403,CI 10316, COLIPA n° B001), Acid Yellow 3 (COLIPA n° : C 54, D&C Yellow N° 10, Quinoline Yellow, E104, Food Yellow 13), Acid Yellow 9 (CI 13015), Acid Yellow 17 (CI 18965), Acid Yellow 23 (COLIPA n° C 29, Covacap Jaune W 1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, CI 15510, D&C Orange 4, COLIPA n° C015), Acid Orange 10 (C.I. 16230; Orange G sodium salt), Acid Orange 11 (CI 45370), Acid Orange 15 (CI 50120), Acid Orange 20 (CI 14600), Acid Orange 24 (BROWN 1;CI 20170;KATSU201;nosodiumsalt;Brown No.201;RESORCIN BROWN;ACID ORANGE 24;Japan Brown 201;D & C Brown No.1), Acid Red 14 (C.I.14720), Acid Red 18 (E124, Red 18; CI 16255), Acid Red 27 (E 123, CI 16185, C-Rot 46, Echtrot D, FD&C Red Nr.2, Food Red 9, Naphtholrot S), Acid Red 33 (Red 33, Fuchsia Red, D&C Red 33, CI 17200), Acid Red 35 (CI C.I.18065), Acid Red 51 (CI 45430, Pyrosin B, Tetraiodfluorescein, Eosin J, lodeosin), Acid Red 52 (CI 45100, Food Red 106, Solar Rhodamine B, Acid Rhodamine B, Red n° 106 Pontacyl Brilliant Pink), Acid Red 73 (CI CI 27290), Acid Red 87 (Eosin, CI 45380), Acid Red 92 (COLIPA n° C53, CI 45410), Acid Red 95 (CI 45425, Erythtosine,Simacid Erythrosine Y), Acid Red 184 (CI 15685), Acid Red 195, Acid Violet 43 (Jarocol Violet 43, Ext. D&C Violet n° 2, C.I. 60730, COLIPA n° C063), Acid Violet 49 (CI 42640), Acid Violet 50 (CI 50325), Acid Blue 1 (Patent Blue, CI 42045), Acid Blue 3 (Patent Blau V, CI 42051), Acid Blue 7 (CI 42080), Acid Blue 104 (CI 42735), Acid Blue 9 (E 133, Patentblau AE, Amidoblau AE, Erioglaucin A, CI 42090, C.I. Food Blue 2), Acid Blue 62 (CI 62045), Acid Blue 74 (E 132, CI 73015), Acid Blue 80 (CI 61585), Acid Green 3 (CI 42085, Foodgreen1), Acid Green 5 (CI 42095), Acid Green 9 (C.I.42100), Acid Green 22 (C.I.42170), Acid Green 25 (CI 61570, Japan Green 201, D&C Green No. 5), Acid Green 50 (Brillantsäuregrün BS, C.I. 44090, Acid Brilliant Green BS, E 142), Acid Black 1 (Black n° 401, Naphthalene Black 10B, Amido Black 10B, CI 20 470, COLIPA n° B15), Acid Black 52 (CI 15711), Food Yellow 8 (CI 14270), Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Die Wasserlöslichkeit der anionischen direktziehenden Farbstoffe kann beispielsweise auf dem folgenden Weg bestimmt werden. 0,1 g des anionischen direktziehenden Farbstoffes werden in ein Becherglas gegeben. Es wird ein Rührfisch hinzugegeben. Dann werden 100 ml Wasser hinzugefügt. Diese Mischung wird auf einem Magnetrührer unter Rühren auf 25 °C erwärmt. Es wird für 60 Minuten gerührt. Danach wird die wässrige Mischung visuell beurteilt. Sind noch ungelöste Reste vorhanden, wird die Wassermenge - beispielsweise in Schritten von 10 ml - erhöht. Es wird solange Wasser zugegeben, bis sich die eingesetzte Farbstoffmenge komplett gelöst hat. Sofern sich die Farbstoff-Wasser-Mischung aufgrund der hohen Intenstität des Farbstoffes nicht visuell beurteilten lässt, wird die Mischung filtriert. Bleibt auf dem Filterpapier ein Anteil an ungelösten Farbstoffen zurück, wird der Löslichkeitsversuch mit einer höheren Wassermenge wiederholt. Lösen sich 0,1 g des anionischen direktziehenden Farbstoffes bei 25 °C in 100 ml Wasser, so liegt die Löslichkeit des Farbstoffes bei 1,0 g/L.

Acid Yellow 1 trägt den Namen 8-Hydroxy-5,7-dinitro-2-naphthalenesulfonsäure Dinatriumsalz und besitzt eine Löslichkeit in Wasser von mindestens 40 g/L (25°C).

Acid Yellow 3 ist ein Gemisch der Natriuimsalze von Mono- und Sisulfonsäuren von 2-(2-Chinolyl)-1H-indene-1,3(2H)-dion und besitzt eine Wasserlöslichkeit von 20 g/L (25 °C).

Acid Yellow 9 ist das Dinatriumsalz der 8-Hydroxy-5,7-dinitro-2-naphthalenesulfonsäure, seine Wasserlöslichkeit liegt oberhalb von 40 g/L (25 °C).

Acid Yellow 23 ist das Trinatriumsalz der4,5-Dihydro-5-oxo-1-(4-sulfophenyl)-4-((4-sulfophenyl)azo)-1H-pyrazole-3-carbonsäure und bei 25 °C gut in Wasser löslich.

Acid Orange 7 ist das Natriumsalz des 4-[(2-Hydroxy-1-naphthyl)azo]benzensulfonats. Seine Wasserlöslichkeit beträgt mehr als 7 g/L (25 °C).

Acid Red 18 ist das Trinatirumsalz von 7-Hydroxy-8-[(E)-(4-sulfonato-1-naphthyl)-diazenyl)]-1,3-naphthalenedisulfonat und besitzt eine sehr hohe Wasserlöslichkeit von mehr als 20 GEw.-%.

Acid Red 33 ist das Diantriumsalz des 5-Amino-4-hydroxy-3-(phenylazo)-naphthalene-2,7-disulphonats, seine Wasserlöslichkeit liegt bei 2,5 g/L (25 °C).

Bei Acid Red 92 handelt es sich um das Dinatriumsalz der 3,4,5,6-Tetrachloro-2-(1 ,4,5,8-tetrabromo-6-hydroxy-3-oxoxanthen-9-yl)benzoesäure, dessen Wasserlöslichkeit mit größer 10 g/L angegeben wird (25 °C).

Acid Blue 9 ist das Dinatriumsalz von 2-({4-[N-ethyl(3-sulfonatobenzyl]amino]phenyl}{4-[(N-ethyl(3-sulfonatobenzyl)imino]-2,5-cyclohexadien-1-ylidene}methyl)-benzenesulfonat und besitzt eine Wasserlöslichkeit von mehr als 20 Gew.-% (25 °C).

In einer weiteren Ausführungsform ist ein erfindungsgemäßes Verfahren ist daher dadurch gekennzeichnet, dass das Mittel (a) mindestens einen direktziehenden Farbstoff (a2) enthält, der ausgewählt ist aus der Gruppe aus Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red, Acid Red 27, Acid Red 33, Acid Red 35, Acid Red 51, Acid Red 52, Acid Red 73, Acid Red 87, Acid Red 92, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Der oder die direktziehenden Farbstoffe können je nach erwünschter Farbintensität in verschiedenen Mengen im Mittel (a) eingesetzt werden. Besonders gute Ergebnisse konnten erhalten werden, wenn das Mittel (a) - bezogen auf das Gesamtgewicht des Mittels (a) - ein oder mehrere direktziehende Farbstoffe in einer Gesamtmenge von 2,0 bis 95,0 Gew.-%, bevorzugt 4,0 bis 70,0 Gew.-%, weiter bevorzugt von 6,0 bis 50,0 Gew.-% und ganz besonders bevorzugt von 8,0 bis 30,0 Gew.-% enthielt.

Weiterhin kann das Mittel (a) als farbgebende Verbindung (a2) auch mindestens einen photochromen oder thermochromen Farbstoff enthalten.

Photochrome Farbstoffe sind Farbstoffe, die auf Bestrahlung mit UV-Licht (Sonnenlicht oder Schwarzlicht) mit einer reversiblen Farbtonänderung reagieren. Dabei verändert das UV-Licht die chemische Struktur der Farbstoffe und damit ihr Absorptionsverhalten (Photochromie).

Thermochrome Farbstoffe sind Farbstoffe, die auf Temperaturänderungen mit einer reversiblen Farbtonänderung reagieren. Dabei verändert die Temperaturänderung die chemische Struktur der Farbstoffe und damit ihr Absorptionsverhalten (Thermochromie).

Das Mittel (a) kann - bezogen auf das Gesamtgewicht des Mittels (a) - ein oder mehrere photochrome Farbstoffe (b) in einer Gesamtmenge von 2,0 bis 95,0 Gew.-%, bevorzugt 4,0 bis 70,0 Gew.-%, weiter bevorzugt von 6,0 bis 50,0 Gew.-% und ganz besonders bevorzugt von 8,0 bis 30,0 Gew.-% enthalten.

Das Mittel (a) kann - bezogen auf das Gesamtgewicht des Mittels (a) - ein oder mehrere thermochrome Farbstoffe (b) in einer Gesamtmenge von 2,0 bis 95,0 Gew.-%, bevorzugt 4,0 bis 70,0 Gew.-%, weiter bevorzugt von 6,0 bis 50,0 Gew.-% und ganz besonders bevorzugt von 8,0 bis 30,0 Gew.-% enthalten.

### Lösungsmittel (a3) im Mittel (a)

Der Einsatz von Lösungsmitteln (a3) hat weiterhin zu sehr guten Ergebnissen geführt. Aus diesem Grund kann das erfindungsgemäße Mittel (a) daher als optionalen Bestandteil (a3) zusätzlich mindestens ein Lösungmittel enthalten.

Als geeignete Lösungsmittel (a3) können beispielsweise Lösungsmittel aus der Gruppe aus 1,2-Propylenglycol, 1,3-Propylenglycol, Ethylenglycol, 1,2- Butylenglycol, Dipropylenglycol, Ethanol, Isopropanol, Diethylenglycolmonoethylether, Glycerin, Phenoxyethanol und Benzylalkohol eingesetzt werden. Der Einsatz von 1,2-Propylenglycol ist ganz besonders bevorzugt.

In einer weiteren ganz besonders bevorzugten Ausführungsform ist ein erfindungsgemäßes Verfahren dadurch gekennzeichnet, dass das Mittel (a) mindestens ein Lösungsmittel (a3) aus der Gruppe aus 1,2-Propylenglycol, 1,3-Propylenglycol, Ethylenglycol, 1,2- Butylenglycol, Dipropylenglycol, Ethanol, Isopropanol, Diethylenglycolmonoethylether, Glycerin, Phenoxyethanol und Benzylalkohol, ganz besonders bevorzugt 1,2-Propylenglycol, enthält.

1,2-Propylenglycol wird alternativ auch als 1,2-Propandiol bezeichnet wird und trägt die CAS-Nummern 57-55-6 [(RS)-1,2-Dihydroxypropan], 4254-14-2 [(R)-1,2-Dihydroxypropan] und 4254-15-3 [(S)-1,2-Dihydroxypropan]. Ethylenglycol wird alternativ auch als 1,2-Ethandiol bezeichnet und trägt die CAS-Nummer 107-21-1.Glycerin wird alternativ auch als 1,2,3-Propantriol bezeichnet und trägt die CAS-Nummer 56-81-5. Phenoxyethanol besitzt die Cas-Nummer 122-99-6.

Alle zuvor beschriebenen Lösungsmittel sind bei verschiedenen Chemikalien-Lieferanten wie beispielsweise Aldrich oder Fluka kommerziell erhältlich.

Durch Verwendung der vorgenannten Lösungsmittel in geeigneten Einsatzmengen kann ein besonders stabiles Mittel (a) erhalten werden, das sich mit dem Mittel (b) besonders schnell und gleichmäßig vermischen lässt. Auch wurden bei Einsatz der geeigneten und bevorzugten Lösungsmittel (a3), insbesondere bei Einsatz von 1,2-Propylenglycol, auf Keratinmaterial Farbergebnisse mit ganz besonders hoher Intensität erhalten.

In einer weiteren bevorzugten Ausführungsform ist ein erfindungsgemäßes Verfahren dadurch gekennzeichnet, dass das Mittel (a) - bezogen auf das Gesamtgewicht des Mittels (a) - ein oder mehrere Lösungsmittel (a3) in einer Gesamtmenge von 1,0 bis 95,0 Gew.-%, bevorzugt 20,0 bis 90,0 Gew.-%, weiter bevorzugt 40,0 bis 85,0 Gew.-% und ganz besonders bevorzugt 60,0 bis 85,0 Gew.-% enthält.

In einer weiteren ganz besonders bevorzugten Ausführungsform ist ein erfindungsgemäßes Verfahren dadurch gekennzeichnet, dass das Mittel (a) - bezogen auf das Gesamtgewicht des Mittels (a) - 1,0 bis 95,0 Gew.-%, bevorzugt 20,0 bis 90,0 Gew.-%, weiter bevorzugt 40,0 bis 85,0 Gew.-% und ganz besonders bevorzugt 60,0 bis 85,0 Gew.-% 1,2-Propylenglycol enthält.

### Konfektionierung des Mittels (a)

Wie bereits zuvor beschrieben, handelt es sich bei dem Mittel (a) um einen Premix, ein Konzentrat oder eine Vor-Mischung, welche als Hauptbestandteile die erfindungswesentlichen Inhaltstoffe (a1) und (a2) enthält. Bevorzugt enthält das Mittel (a) als dritten optionalen Inhaltstoff (a3) mindestens ein Lösungsmittel.

Ohne auf diese Theorie beschränkt zu sein, wird vermutet, dass die farbgebenden Verbindungen (a2) - insbesondere wenn es sich hierbei um Pigmente handelt - und die aminofunktionalisierten Silikonpolymere (a1) miteinander interagieren können. Diese Interaktion scheint vor allem in wässriger Umgebung bzw. in wasserhaltigem Milieu stattzufinden. Eine Vermutung ist, dass zwischen der jeweiligen Oberfläche des Pigments und den Aminogruppen des Silikonpolymers eine Wechselwirkung auftritt, wobei das Wasser möglicherweise als Protonendonator oder Protonenakzeptor fungieren könnte.

Gestützt wird diese Vermutung durch Beobachtungen, die gezeigt haben, dass bei einer Formulierung, welche neben Aminosilikon (a1) und Pigment (a2) auch die Bestandteile der Trägerformulierung, d.h. Wasser (b1) und Fettbestandteile (b2) in höheren Konzentrationen enthielt, nach einigen Tagen die Abscheidung einer harzigen Substanz beobachtet werden konnte. Wendete man diese Formulierung nach mehrtägiger Lagerzeit im Färbeversuch an, so wurden lediglich Färbungen mit sehr geringer Farbintensität erhalten.

Überraschenderweise blieb ein einsprechend wasserfreies Mittel (a), welches in Form des Premixes bzw. Konzentrats formuliert war und kein Wasser (b1) und keine Fettbestandteile (b2) enthielt ohne Verklumpung bzw. Harzabscheidung stabil. Aus diesem Grund hat es sich als ganz besonders bevorzugt erwiesen, den Wassergehalt im Mittel (a) entsprechend gering zu wählen. Mit Mitteln (a), welche einen Wassergehalt von maximal 10 Gew.-% besaßen, wurden bereits sehr intensive Farbergebnisse erhalten. Die Lagerstabilität und die Färbeleistung konnten jedoch noch weiter verbessert werden, wenn der Wassergehalt im Mittel (a) auf einen Maximalwert von höchstens 5,0 Gew.-%, weiter bevorzugt höchstens 2,5 Gew.-%, und ganz besonders bevorzugt von höchstens 1,0 Gew.-% reduziert wurde. In diesem Zusammenhang ist der Wassergehalt auf das Gesamtgewicht des Mittels (a) bezogen.

Ein erfindungsgemäßes Verfahren wird daher dadurch gekennzeichnet, dass das Mittel (a) - bezogen auf das Gesamtgewicht des Mittels (a) - weniger als 10,0 Gew.-%, bevorzugt weniger als 5,0 Gew.-%, weiter bevorzugt weniger als 2,5 Gew.-% und ganz besonders bevorzugt weniger als 1,0 Gew.-% Wasser enthält.

Mit anderen Worten ist ein erfindungsgemäßes Verfahren dadurch gekennzeichnet, dass das Mittel (a) - bezogen auf das Gesamtgewicht des Mittels (a) - einen Wassergehalt zwischen 0 und 10,0 Gew.-%, bevorzugt zwischen 0 und 5,0 Gew.-%, weiter bevorzugt zwischen 0 und 2,5 Gew.-% und ganz besonders bevorzugt zwischen 0 und 1,0 Gew.-% besitzt.

Der erfindungsgemäße Premix bzw. das Mittel (a) enthält als Hauptbestandteile die vorgenannten Inhaltstoffe (a1), (a2) und optional (a3), die besonders bevorzugt in den entsprechenden hohen Mengen im Mittel (a) eingesetzt werden.

Prinzipiell kann der Premix oder das Konzentrat optional auch noch weitere andere Inhaltsstoffe enthalten, welche von den Bestandteilen (a1), (a2) sowie ggf. (a3) verschieden sind. Bei diesen anderen Bestandteilen kann es sich beispielsweise um Konservierungsmittel, Parfums oder Verdicker handeln. Es konnten jedoch ganz besonders gute Lagerstabiltitäten erhalten werden, wenn das Mittel (a) in einem wesentlichen Anteil aus den Inhaltsstoffen (a1), (a2) und optional (a3) bestand. Als besonders vorteilhaft im Hinblick auf die erfindungsgemäße Aufgabenstellung hat es sich deshalb erwiesen, wenn die Bestandteile (a1), (a2) und (a3) - bezogen auf das Gesamtgewicht des Mittels (a) - zusammen einen Gewichtsanteil von mindestens 70,0 Gew.-%, bevorzugt von mindestens 80,0 Gew.-%, weiter bevorzugt von mindestens 90,0 Gew.-% und ganz besonders bevorzugt von mindestens 95,0 Gew.-% ausmachten.

Anders ausgedrückt war es ganz besonders vorteilhaft, wenn das Mittel (a) weitere Inhaltsstoffe, die von den Bestandteilen (a1), (a2) und (a3) verschieden waren, nur zu einem Gewichtsanteil von höchtens 30,0 Gew.-%, bevorzugt zu höchstens 20,0 Gew.-%, und ganz besonders bevorzugt nur zu 10,0 Gew.-% enthält.

In einer weiteren explizit ganz besonders bevorzugten Ausführungsform ist ein erfindungsgemäßes Verfahren dadurch gekennzeichnet, dass die Bestandteile (a1), (a2) und (a3) - bezogen auf das Gesamtgewicht des Mittels (a) - zusammen einen Gewichtsanteil von mindestens 70,0 Gew.-%, bevorzugt von mindestens 80,0 Gew.-%, weiter bevorzugt von mindestens 90,0 Gew.-% und ganz besonders bevorzugt von mindestens 98,0 Gew.-% besitzen.

Sind die Lösungsmittel (a3) als optionale Bestanteilte nicht im Mittel (a) enthalten, so befinden sich nur die Inhaltsstoffe (a1) und (a2) als Hauptbestandteile im Mittel (a). Im Rahmen dieser Ausführungsform ist es von Vorteil, wenn das Mittel (a) weitere Inhaltsstoffe, die von den Bestandteilen (a1) und (a2) verschieden sind, nur zu einem Gewichtsanteil von höchtens 30,0 Gew.-%, bevorzugt zu höchstens 20,0 Gew.-%, und ganz besonders bevorzugt nur zu 10,0 Gew.-% enthält.

In einer weiteren besonders bevorzugten Ausführungsform ist ein erfindungsgemäßes Verfahren dadurch gekennzeichnet, dass die Bestandteile (a1) und (a2) - bezogen auf das Gesamtgewicht des Mittels (a) - zusammen einen Gewichtsanteil von mindestens 70,0 Gew.-%, bevorzugt von mindestens 80,0 Gew.-%, weiter bevorzugt von mindestens 90,0 Gew.-% und ganz besonders bevorzugt von mindestens 95,0 Gew.-% besitzen.

### Mittel (b)

In Schritt (2) des erfindungsgemäßen Verfahren wird das Mittel (b) bereitgestellt. Beispielsweise kann das Mittel (b) in einer Verpackungseinheit oder einem Container vorliegen und auf diese Weise dem Anwender zur Verfügung gestellt werden. Bei dem Container kann es sich beispielsweise um ein Sachet, eine Flasche, eine Dose, einen Tiegel oder auch ein anderes für kosmetische Formulierungen geeignetes Behältnis handeln.

Das Mittel (b) stellt eine Trägerformulierung oder auch Basisformulierung dar und ist gekennzeichnet durch seinen genau eingestellten Wasserhalt (b1) und seinen Gehalt an Fettbestandteil(en) (b2).

Mlt Abmischung der Mittel (a) und (b) wird der bevorzugt hochkonzentrierte und wasserarme Premix in eine anwendungsbereite Form überführt, die dann auf dem Keratinmaterial appliziert werden kann.

### Wassergehalt (b1) im Mittel (b)

Das Mittel (b) ist dadurch gekennzeichnet, dass es entweder kein Wasser enthält, d.h. dass sein Wassergehalt - bezogen auf das Gesamtgewicht des Mittels (b) - bei 0 Gew.-% liegt, oder aber dass es einen reduzierten Wassergehalt von maximal 50 Gew.-% besitzt.

Im Rahmen der zu dieser Erfindung führenden Arbeiten wurde gefunden, dass der Wassergehalt des Mittels (b) einen wesentlichen Einfluss auf das Griffgefühl des Keratinmaterials bzw. des Haares nimmt.

Wurde beispielsweise eine Formulierung, die neben Fettbestandteil (b2) einen sehr hohen Wassergehalt besaß, mit dem erfindungsgemäßen Mittel (a) germischt, so konnten zwar Färbungen mit hoher Farbintensität erhalten werden, das Griffgefühl der auf diese Weise gefärbten Keratinfasern war jedoch sehr schlecht. Unter einem schlechten Griffgefühl muss in diesem Zusammenhang verstanden werden, dass die Keratinfasern, insbesondere die Haare, sich stumpf, rauh und hart bzw. widerspenstig anfühlen.

Wurde das erfindungsgemäße Mittel (a) hingegen mit einem erfindungsgemäßen Mittel (b) vermischt, so waren die gefärbten Haare viel weicher und fühlten sich angenehmer und nicht stumpf an.

Um sowohl die Farbintenstiät als auch das Griffgefühl der gefärbten Keratinfasern zu optimieren, wird das Mittel (b) daher ganz besonders bevorzugt auf einen ganz bestimmten Wassergehalt eingestellt. Die allerbesten Ergebnisse konnten erhalten werden, wenn das Mittel (b) - bezogen auf das Gesamtgewicht des Mittels (b) - 0 bis 45 Gew.-%, bevorzugt 5 bis 40 Gew.-%, weiter bevorzugt 10 bis 35 Gew.-% und ganz besonders bevorzugt 15 bis 35 Gew.-% Wasser (b1) enthält.

In einer weiteren explizit ganz besonders bevorzugten Ausführungsform ist ein erfindungsgemäßes Verfahren dadurch gekennzeichnet, dass das Mittel (b) - bezogen auf das Gesamtgewicht des Mittels (b) - 0 bis 45 Gew.-%, bevorzugt 5 bis 40 Gew.-%, weiter bevorzugt 10 bis 35 Gew.-% und ganz besonders bevorzugt 15 bis 35 Gew.-% Wasser (b1) enthält.

### Fettbestandteile (b2) im Mittel (b)

Kennzeichnend für das Mittel (b) ist weiterhin sein Gehalt an mindestens einem Fettbestandteil (b2). Es hat sich herausgestellt, dass der Einsatz mindestens eines Fettbestandteils dazu führt, dass das Mittel (b) in Form einer Emulsion vorliegt, welche eine besonders gute und schnelle Vermischung mit dem Mittel (a) ermöglicht.

Bei den Fettbestandteilen handelt es sich um hydrophobe Substanzen, die in Gegenwart von Wasser unter Ausbildung von Mizell-Systemen Emulsionen formen können.

Unter "Fettbestandteilen" werden im Sinne der Erfindung organische Verbindungen mit einer Löslichkeit in Wasser bei Raumtemperatur (22 °C) und atmosphärischem Druck (760 mmHg) von weniger als 1 Gew.-%, bevorzugt von weniger als 0,1 Gew.-% verstanden. Unter die Definition der Fettbestandteile fallen explizit nur ungeladene (d.h. nichtionische) Verbindungen. Fettbestandteile besitzen mindestens eine gesättigte oder ungesättigte Alkylgruppe mit mindestens 12 C-Atomen. Das Molgewicht der Fettbestandteile liegt bei maximal 5000 g/mol, bevorzugt bei maximal 2500 g/mol und besonders bevorzugt bei maximal 1000 g/mol. Bei den Fettbestandteilen handelt es sich weder um polyoxyalkylierte noch um polyglycerylierte Verbindungen.

Ganz besonders bevorzugt werden die im Mittel (b) enthaltenen Fettbestandteile (b2) ausgewählt aus der Gruppe der Esteröle, der C₁₂-C₃₀-Fettalkohole, der C₁₂-C₃₀-Fettsäuretriglyceride, der C₁₂-C₃₀-Fettsäuremonoglyceride, der C₁₂-C₃₀-Fettsäurediglyceride und/oder der Kohlenwasserstoffe. Sinne der vorliegenden Erfindung werden explizit nur nichtionische Substanzen als Fettbestandteile betrachtet. Geladene Verbindungen wie beispielsweise Fettsäuren und ihre Salze werden nicht als Fettbestandteil verstanden.

Im Rahmen einer weiteren bevorzugten Ausführungsform ist ein erfindungsgemäßes Verfahren dadurch gekennzeichnet, dass das Mittel (b) einen oder mehrere Fettbestandteile (b2) aus der Gruppe der Esteröle, C₁₂-C₃₀-Fettalkohole, der C₁₂-C₃₀-Fettsäuretriglyceride, der C₁₂-C₃₀-Fettsäure-monoglyceride, der C₁₂-C₃₀-Fettsäurediglyceride und/oder der Kohlenwasserstoffe enthält.

Unter Esterölen werden erfindungsgemäße die Ester von C₆-C₃₀-Alkancarbonsäuren mit aliphatischen C₂-C₃₀-Alkoholen verstanden. Ganz besonders wird bei der Veresterung zum Esteröl ein aliphatischer C2-C30-Monoalkohol eingesetzten werden. Aliphatische C₂-C₃₀-Monoalkohole sind Verbindungen, die nur eine Hydroxygruppe besitzen.

In einer weiteren ganz besonders bevorzugten Ausführungsform ist ein erfindungsgemäßes Verfahren dadurch gekennzeichnet, dass das Mittel (b) mindestens einen Fettbestandteil (b2) aus der Gruppe der Ester einer C₆-C₃₀-Alkancarbonsäure mit einem aliphatischen C₂-C₃₀-Monoalkohol enthält.

Bevorzugt sind die Monoester von C₆-C₃₀-Alkancarbonsäuren mit aliphatischen C₂-C₂₄-Alkoholen. Beispiele für eingesetzte Fettsäurenanteile in den Estern sind Capronsäure, Caprylsäure, 2--Ethyl-hexansäure, Caprinsäure, Laurinsäure, Isotridecansäure, My-ri-stinsäure, Palmitinsäure, Palmitoleinsäure, Stearinsäure, Isostearinsäure, Öl-säure, Elaidinsäure, Petroselinsäure, Li-nolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosyn-these oder der Dimerisierung von ungesättigten Fettsäuren anfallen. Beispiele für die aliphatischen C₂-C₃₀-Alkohole in den Esterölen sind Isopropylalkohol, Capronalkohol, Caprylalkohol, 2-Ethylhexylalkohol, Caprinalkohol, Laurylalkohol, Isotridecylalko-hol, Myristylalkohol, Cetylalkohol, Palmoleylalkohol, Stearylalkohol, Isostearylalkohol, Oleylalkohol, Elaidylalkohol, Petroselinylalkohol, Li-nolylalkohol, Linolenylalkohol, Elaeostearylalkohol, Arachylalkohol, Gadoleylalkohol, Behenylalkohol, Erucylalkohol und Brassidylalkohol sowie deren technische Mi-schungen, die z.B. bei der Hochdruckhydrierung von technischen Methylestern auf Basis von Fetten und Ölen oder Aldehyden aus der Roelen'schen Oxosynthese sowie als Monomer-fraktion bei der Dimeri-sierung von ungesättigten Fettalkoholen anfallen.

In einer weiteren ganz besonders bevorzugten Ausführungsform ist ein erfindungsgemäßes Verfahren dadurch gekennzeichnet, dass das Mittel (b) mindestens einen Fettbestandteil (b2) enthält, der durch Veresterung einer C6-C30-Alkancarbonsäure aus der Gruppe aus Capronsäure, Caprylsäure, 2--Ethyl-hexansäure, Caprinsäure, Laurinsäure, Isotridecansäure, My-ri-stinsäure, Palmitinsäure, Palmitoleinsäure, Stearinsäure, Isostearinsäure, Öl-säure, Elaidinsäure, Petroselinsäure, Li-nolsäure, Linolensäure, Elaeostearinsäure, Arachinsäure, Gadoleinsäure, Behensäure und Erucasäure mit einem C2-C30-Alkohol aus der Gruppe aus Isopropylalkohol, Capronalkohol, Caprylalkohol, 2-Ethylhexylalkohol, Caprinalkohol, Laurylalkohol, Isotridecylalko-hol, Myristylalkohol, Cetylalkohol, Palmoleylalkohol, Stearylalkohol, Isostearylalkohol, Oleylalkohol, Elaidylalkohol, Petroselinylalkohol, Li-nolylalkohol, Linolenylalkohol, Elaeostearylalkohol, Arachylalkohol, Gadoleylalkohol, Behenylalkohol, Erucylalkohol und Brassidylalkohol erhalten wird.

Erfindungsgemäß besonders bevorzugte Esteröle sind Stearinsäure-2-ethylhexylester (Cetiol^{®} 868), Isopropylmyristat (Rilanit^{®} IPM), Isononansäure-C16-18-alkylester (Cetiol^{®} SN), 2-Ethylhexylpalmitat (Cegesoft^{®} 24), Cetyloleat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).

Ein explizit ganz besonders bevorzugtes Esteröl ist Erfindungsgemäß besonders bevorzugte Esteröle sind Stearinsäure-2-ethylhexylester (Cetiol^{®} 868).

In einer weiteren ganz besonders bevorzugten Ausführungsform ist ein erfindungsgemäßes Verfahren dadurch gekennzeichnet, dass das Mittel (b) mindestens einen Fettbestandteil (b2) aus der Gruppe aus Stearinsäure-2-ethylhexylester, Isopropylmyristat, Isononansäure-C16-18-alkylester, 2-Ethylhexylpalmitat, Cetyloleat, Kokosfettalkoholcaprinat, Kokosfettalkoholcaprylat, n-Butylstearat, Oleylerucat, Isopropylpalmitat, Oleyloleat, Laurinsäurehexylester, Myristylmyristat, Cetearyl Isononanoate und Ölsäuredecylester enthält.

Bei den C₁₂-C₃₀-Fettalkoholen kann es sich um gesättigte, ein- oder mehrfach ungesättigte, lineare oder verzweigte Fettalkohole mit 12 bis 30 C-Atomen handeln.

Beispiele für bevorzugte lineare, gesättigte C₁₂-C₃₀-Fettalkohole sind Dodecan-1-ol (Dodecylalkohol, Laurylalkohol), Tetradecan-1-ol (Tetradecylalkohol, Myristylalkohol), Hexadecan-1-ol (Hexadecylalkohol, Cetylalkohol, Palmitylalkohol), Octadecan-1-ol (Octadecylalkohol, Stearylalkohol), Arachylalkohol (Eicosan-1-ol), Heneicosylalkohol (Heneicosan-1-ol) und/oder Behenylalkohol (Docosan-1-ol).

Bevorzugte lineare, ungesättigte Fettalkohole sind (9*Z*)-Octadec-9-en-1-ol (Oleylalkohol), (9*E*)-Octadec-9-en-1-ol (Elaidylalkohol), (9*Z*,12*Z*)-Octadeca-9,12-dien-1-ol (Linoleylalkohol), (9*Z*,12*Z*,15*Z*)-Octadeca-9,12,15-trien-1-ol (Linolenoylalkohol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Erucylalkohol ((13Z)-Docos-13-en-1-ol) und/oder Brassidylalkohol ((13*E*)-Docosen-1-ol).

Geeignete Vertreter für verzweigte Fettalkohole sind 2-Octyl-dodecanol, 2-Hexyl-Dodecanol und/oder 2-Butyl-dodecanol.

Im Rahmen einer Ausführungsform wurden weiterhin gute Ergebnisse erhalten, wenn das Mittel (b) einen oder mehrere C₁₂-C₃₀-Fettalkohole aus der Gruppe aus Dodecan-1-ol (Dodecylalkohol, Laurylalkohol), Tetradecan-1-ol (Tetradecylalkohol, Myristylalkohol), Hexadecan-1-ol (Hexadecylalkohol, Cetylalkohol, Palmitylalkohol), Octadecan-1-ol (Octadecylalkohol, Stearylalkohol), Arachylalkohol (Eicosan-1-ol), Heneicosylalkohol (Heneicosan-1-ol), Behenylalkohol (Docosan-1-ol), (9*Z*)-Octadec-9-en-1-ol (Oleylalkohol), (9*E*)-Octadec-9-en-1-ol (Elaidylalkohol), (9*Z*, 12*Z*)-Octadeca-9, 12-dien-1-ol (Linoleylalkohol), (9*Z*,12*Z*,15*Z*)-Octadeca-9,12,15-trien-1-ol (Linolenoylalkohol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Erucylalkohol ((13*Z*)-Docos-13-en-1-ol), Brassidylalkohol ((13*E*)-Docosen-1-ol) 2-Octyl-dodecanol, 2-Hexyl-Dodecanol und/oder 2-Butyl-dodecanol enthält.

Im Rahmen einer weiteren bevorzugten Ausführungsform ist ein erfindungsgemäßes Verfahren dadurch gekennzeichnet, dass das zweite Mittel (b) einen oder mehrere C₁₂-C₃₀-Fettalkohole (b2) aus der Gruppe aus
Dodecan-1-ol (Dodecylalkohol, Laurylalkohol),
Tetradecan-1-ol (Tetradecylalkohol, Myristylalkohol),
Hexadecan-1-ol (Hexadecylalkohol, Cetylalkohol, Palmitylalkohol),
Octadecan-1-ol (Octadecylalkohol, Stearylalkohol),
Arachylalkohol (Eicosan-1-ol),
Heneicosylalkohol (Heneicosan-1-ol),
Behenylalkohol (Docosan-1-ol),
(9*Z*)-Octadec-9-en-1-ol (Oleylalkohol),
   (9*E*)-Octadec-9-en-1-ol (Elaidylalkohol),
   (9*Z*,12*Z*)-Octadeca-9,12-dien-1-ol (Linoleylalkohol),
(9*Z*,12*Z*,15*Z*)-Octadeca-9,12,15-trien-1-ol (Linolenoylalkohol),
Gadoleylalkohol ((9*Z*)-Eicos-9-en-1-ol),
Arachidonalkohol ((5*Z*,8*Z*,11*Z*,14*Z*)-Eicosa-5,8,11,14-tetraen-1-ol),
Erucylalkohol ((13*Z*)-Docos-13-en-1-ol),
Brassidylalkohol ((13*E*)-Docosen-1-ol),
2-Octyl-dodecanol,
2-Hexyl-Dodecanol und/oder
2-Butyl-dodecanol enthält.

Weiterhin als gut geeigneten Fettbestandteil (b2) kann das Mittel (b) auch mindestens ein C₁₂-C₃₀-Fettsäuretriglycerid, der C₁₂-C₃₀-Fettsäuremonoglycerid und/oder C₁₂-C₃₀-Fettsäurediglycerid enthalten. Unter einem C₁₂-C₃₀-Fettsäuretriglycerid wird im Sinne der vorliegenden Erfindung der Triester des dreiwertigen Alkohols Glycerin mit drei Äquivalenten Fettsäure verstanden. Dabei können sowohl strukturgleiche als auch unterschiedliche Fettsäuren innerhalb eines Triglyceridmoleküls an den Esterbildungen beteiligt sein.

Unter Fettsäuren sind erfindungsgemäß gesättigte oder ungesättigte, unverzweigte oder verzweigte, unsubstituierte oder substituierte C₁₂-C₃₀-Carbonsäuren zu verstehen. Ungesättigte Fettsäuren können einfach oder mehrfach ungesättigt sein. Bei einer ungesättigten Fettsäure kann bzw. können deren C-C-Doppelbindung(en) die Cis- oder Trans-Konfiguration aufweisen.

Es zeichnen sich die Fettsäuretriglyceride durch besondere Eignung aus, bei welchen mindestens eine der Estergruppen ausgehend von Glycerin mit einer Fettsäure ausgebildet wird, die ausgewählt wird aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(*Z*)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)- Tetracos-15-ensäure].

Die Fettsäuretriglyceride können auch natürlichen Ursprungs sein. Die in Sojaöl, Erdnußöl, Olivenöl, Sonnenblumenöl, Macadamianussöl, Moringaöl, Aprikosenkernöl, Marulaöl und/oder gegebenenfalls gehärtetem Rizinusöl vorkommenden Fettsäure-Triglyceride bzw. deren Gemische sind zum Einsatz im erfindungsgemäßen Produkt besonders geeignet.

Unter einem C₁₂-C₃₀-Fettsäuremonoglycerid wird der Monoester des dreiwertigen Alkohols Glycerin mit einem Äquivalent Fettsäure verstanden. Hierbei kann entweder die mittlere Hydroxygruppe des Glycerins oder die endständige Hydroxygruppe des Glycerins mit der Fettsäure verestert sein.

Es zeichnen sich die C₁₂-C₃₀-Fettsäuremonoglycerid durch besondere Eignung aus, bei welchen eine Hydroxygruppe des Glycerins mit einer Fettsäure verestert wird, wobei die Fettsäuren ausgewählt sind aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(*Z*)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9*Z*, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] oder Nervonsäure [(15Z)- Tetracos-15-ensäure].

Unter einem C₁₂-C₃₀-Fettsäurediglycerid wird der Diester des dreiwertigen Alkohols Glycerin mit zwei Äquivalenten Fettsäure verstanden. Hierbei können entweder die mittlere und eine endständige Hydroxygruppe des Glycerins mit zwei Äquivalenten Fettsäure verestert sein, oder aber beide endständigen Hydroxygruppen des Glycerins sind mit jeweils einer Fettsäure verestert. Das Glycerin kann hierbei sowohl mit zwei strukturgleichen als auch mit zwei unterschiedlichen Fettsäuren verestert sein.

Es zeichnen sich die Fettsäurediglyceride durch besondere Eignung aus, bei welchen mindestens eine der Estergruppen ausgehend von Glycerin mit einer Fettsäure ausgebildet wird, die ausgewählt wird aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(*Z*)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)-Tetracos-15-ensäure].

Ganz besonders gute Ergebnisse wurden erhalten, wenn die Zusammensetzung (B) mindestens ein C₁₂-C₃₀-Fettsäuremonoglycerid enthielt, welches ausgewählt ist aus den Monoestern von Glycerin mit einem Äquivalent Fettsäure aus der Gruppe aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)- Tetracos-15-ensäure].

Im Rahmen einer weiteren Ausführungsform ist ein erfindungsgemäßes Verfahren dadurch gekennzeichnet, dass das zweite Mittel (b) mindestens ein C₁₂-C₃₀-Fettsäuremonoglycerid (b2) enthält, welches ausgewählt ist aus den Monoestern von Glycerin mit einem Äquivalent Fettsäure aus der Gruppe aus Dodecansäure, Tetradecansäure, Hexadecansäure, Tetracosansäure, Octadecansäure, Eicosansäure und/oder Docosansäure.

Kohlenwasserstoffe sind ausschließlich aus den Atomen Kohlenstoff und Wasserstoff bestehende Verbindungen mit 8 bis 80 C-Atomen. Bevorzugt sind in diesem Zusammenhang insbesondere aliphatische Kohlenwasserstoffe wie beispielsweise Mineralöle, flüssige Paraffinöle (z.B. Paraffinium Liquidum oder Paraffinum Perliquidum), Isoparaffinöle, halbfeste Paraffinöle, Paraffinwachse, Hartparaffin (Paraffinum Solidum), Vaseline und Polydecene.

Als geeignet haben sich in diesem Zusammenhang flüssige Paraffinöle (Paraffinum Liquidum und Paraffinium Perliquidum) erwiesen. Ganz besonders bevorzugt handelt es sich bei dem Kohlenwasserstoff um Paraffinum Liquidum, auch Weißöl genannt. Bei Paraffinum Liquidum handelt es sich um ein Gemisch gereinigter, gesättigter, aliphatischer Kohlenwasserstoffe, das größtenteils aus Kohlenwasserstoffketten mit einer C-Kettenverteilung von 25 bis 35 C-Atomen besteht.

Gute Ergebnisse wurden ebenfalls erhalten, wenn das Mittel (b) mindestens einen Kohlenwasserstoff (b2) enthielt, der ausgewählt ist aus der Gruppe der Mineralöle, der flüssige Paraffinöle, Isoparaffinöle, halbfeste Paraffinöle, Paraffinwachse, Hartparaffin (Paraffinum Solidum), Vaseline und Polydecene.

Explizit ganz besonders gute Ergebnisse konnten auch erhalten werden, wenn das erfindungsgemäße Mittel mindestens einen Fettbestandteil (b2) aus der Gruppe der Ester-Öle enthielt.

Auch durch Wahl der geeigneten Einsatzmengen an Fettbestandteil (b2) im Mittel (b) kann die Farbintensität der durch das erfindungsgemäße Verfahren erhaltenen Färbung weiter optimiert und das Griffgefühl des Keratinmaterials weiter verbessert werden. Aus diesem Grund hat es sich als ganz besonders bevorzugt erwiesen, ein oder mehrere Esteröle, C₁₂-C₃₀-Fettsäuremono-, C₁₂-C₃₀-Fettsäuredi- und/oder C₁₂-C₃₀-Fettsäuretriglyceride und/oder Kohlenwasserstoffe (b2) in ganz bestimmten Mengenbereichen im Mittel (b) einzusetzen.

Es hat sich als ganz besonders bevozugt erwiesen, wenn das Mittel (b) - bezogen auf das Gesamtgewicht des Mittels (b) - einen oder mehrere Fettbestandteile (b2) in einer Gesamtmenge von 30 bis 99 Gew.-%, bevorzugt von 40 bis 97 Gew.-%, weiter bevorzugt von 50 bis 95 Gew.-% und ganz besonders bevorzug tvon 60 bis 90 Gew.-% enthält.

Im Rahmen einer ganz besonders bevorzugten Ausführungsform ist ein erfindungsgemäßes Verfahren dadurch gekennzeichnet, das Mittel (b) - bezogen auf das Gesamtgewicht des Mittels (b) - einen oder mehrere Fettbestandteile in einer Gesamtmenge von 30 bis 99 Gew.-%, bevorzugt von 40 bis 97 Gew.-%, weiter bevorzugt von 50 bis 95 Gew.-% und ganz besonders bevorzug tvon 60 bis 90 Gew.-% enthält.

Im Rahmen einer ganz besonders bevorzugten Ausführungsform ist ein erfindungsgemäßes Verfahren dadurch gekennzeichnet, das Mittel (b) - bezogen auf das Gesamtgewicht des Mittels (b) - einen oder mehrere Esteröle (b2) in einer Gesamtmenge von 30 bis 99 Gew.-%, bevorzugt von 40 bis 97 Gew.-%, weiter bevorzugt von 50 bis 95 Gew.-% und ganz besonders bevorzug tvon 70 bis 90 Gew.-% enthält.

### Tenside im Mittel (b)

Weiterhin kann es sich als bevorzugt erweisen, im Mittel (b) weiterhin mindestens ein Tensid einzusetzen. Im Rahmen einer weiteren Ausführungsform enthält das Mittel (b) deshalb zusätzlich mindestens ein Tensid.

Im Rahmen einer weiteren besonders bevorzugten Ausführungsform ist ein erfindungsgemäßes Verfahren dadurch gekennzeichnet, dass das Mittel (b) mindestens ein Tensid, enthält.

Unter dem Begriff Tenside (T) werden grenzflächenaktive Substanzen, die an Ober- und Grenzflächen Adsorptionsschichten bilden oder in Volumenphasen zu Mizell-Kolloiden oder lyotropen Mesophasen aggregieren können, verstanden. Man unterscheidet anionische Tenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wässriger Lösung stark hydratisiert sind.

Im Rahmen einer ganz besonders bevorzugten Ausführungsform ist ein erfindungsgemäßes Verfahren dadurch gekennzeichnet, dass das Mittel (b) mindestens ein nichtionisches Tensid (b3) enthält.

Nichtionische Tenside enthalten beipsielsweise als hydrophile Gruppe eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 6 bis 30 C-Atomen, die Fettalkoholpolyglykolether bzw. die Fettalkoholpolypropylenglykolether bzw. gemischte Fettalkoholpolyether,
- Anlagerungsprodukte von 2 bis 50 Mol Ethylenoxid und/oder 0 bis 5 Mol Propylenoxid an lineare und verzweigte Fettsäuren mit 6 bis 30 C-Atomen, die Fettsäurepolyglykolether bzw. die Fettsäurepolypropylenglykolether bzw. gemischte Fettsäurepolyether,
- Anlagerungsprodukte von 2 bis 50 Mol Ethylenoxid und/oder 0 bis 5 Mol Propylenoxid an lineare und verzweigte Alkylphenole mit 8 bis 15 C-Atomen in der Alkylgruppe, die Alkylphenolpolyglykolether bzw. die Alkylpolypropylenglykolether, bzw. gemischte Alyklphenolpolyether,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol^{®} - Typen (Cognis),
- alkoxilierte Triglyceride,
- alkoxilierte Fettsäurealkylester der Formel (Tnio-1)

   R¹CO-(OCH₂CHR²)_{w}OR³ (Tnio-1)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
   - Aminoxide,
   - Hydroxymischether, wie sie beispielsweise in der DE-OS 19738866 beschrieben sind,
   - Sorbitanfettsäureester und Anlagerungeprodukte von Ethylenoxid an Sorbitanfettsäureester wie beispielsweise die Polysorbate,
   - Zuckerfettsäureester und Anlagerungsprodukte von Ethylenoxid an Zuckerfettsäureester,
   - Anlagerungsprodukte von Ethylenoxid an Fettsäurealkanolamide und Fettamine,
   - Zuckertenside vom Typ der Alkyl- und Alkenyloligoglykoside gemäß Formel (E4-II),

      R⁴O-[G]ₚ (Tnio-2)

      in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (Tnio-2) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹⁵ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.
   - Zuckertenside vom Typ der Fettsäure-N-alkylpolyhydroxyalkylamide, ein nichtionisches Tensid der Formel (Tnio-3),

      R⁵CO-NR⁶-[Z] (Tnio-3)

      in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (Tnio-4) wiedergegeben werden:

      R⁷CO-(NR⁸) -CH₂ - [CH(OH)]₄ - CH₂OH (Tnio-4)

      Vorzugsweise werden als Fettsäure-N-alkylpolyhydroxyalkylamide Glucamide der Formel (Tnio-4) eingesetzt, in der R⁸ für Wasserstoff oder eine Alkylgruppe steht und R⁷CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Li-nolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel (Tnio-4), die durch reduktive Aminierung von Glucose mit Methylamin und anschließende Acylierung mit Laurinsäure oder C12/14-Kokosfettsäure beziehungsweise einem entsprechenden Derivat erhalten werden. Weiterhin können sich die Polyhydroxyalkylamide auch von Maltose und Palatinose ableiten.

Die Zuckertenside können in den erfindungsgemäß verwendeten Mitteln bevorzugt in Mengen von 0,1 - 20 Gew.-%, bezogen auf das gesamte Mittel, enthalten sein. Mengen von 0,5 - 15 Gew.-% sind bevorzugt, und ganz besonders bevorzugt sind Mengen von 0,5 - 7,5 Gew.-%.

Weitere typische Beispiele für nichtionische Tenside sind Fettsäureamidpolyglycolether, Fettaminpolyglycolether, Mischether bzw. Mischformale, Proteinhydrolysate (insbesondere pflanzliche Produkte auf Weizenbasis) und Polysorbate.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure sowie die Zuckertenside erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Besonders gute Ergebnisse wurden erhalten, wenn im erfindungsgemäßen Verfahren ein Mittel (b) eingesetzt wurde, welche mindestens einen ethoxylierten Fettalkohol mit einem Ethoxylierungsgrad von 80 bis 120 enthielt.

In einer weiteren ganz besonders bevorzugten Ausführungsform ist ein erfindungsgemäßes Verfahren dadurch gekennzeichnet, dass das Mittel (b) mindestens ein nichtionisches Tensid der Formel (T-I) enthält, 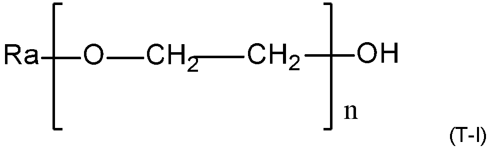
worin Ra für eine gesättigte oder ungesättigte, unverzweigte oder verzweigte C₈-C₂₄-Alkylgruppe, bevorzugt für eine gesättigte, unverzweigte C₁₆- bis C₁₈- Alkylgruppe, steht und
n für eine ganze Zahl von 80 bis 120, bevorzugt für eine ganze Zahl von 90 bis 110 und besonders bevorzugt für die Zahl 100, steht.

Ein besonders gut geeignetes nichtionisches Tensid dieses Typs trägt den Handelsnamen Brij S 100 bzw. Brij S 100 PA SG. Hierbei handelt es sich um Stearylalkohol, ethoxyliert mit 100 EO, der von der Firma Croda kommerziell erhältlich ist und die CAS-Nummer 9005-00-9 trägt.

Weiterhin ganz besonders gute Ergebnisse wurden erhalten, wenn im erfindungsgemäßen Verfahren ein Mittel (b) eingesetzt wurde, welche mindestens einen ethoxylierten Fettalkohole mit einem Ethoxylierungsgrad von 10 bis 40 enthielt.

In einer weiteren ganz besonders bevorzugten Ausführungsform ist ein erfindungsgemäßes Verfahren dadurch gekennzeichnet, dass das Mittel (b) mindestens ein nichtionisches Tensid der Formel (T-II) enthält, 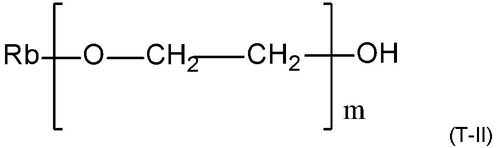 worin
- Rb: für eine gesättigte oder ungesättigte, unverzweigte oder verzweigte C₈-C₂₄-Alkylgruppe, bevorzugt für eine gesättigte, unverzeigte C₁₆- bis C₁₈- Alkylgruppe, steht und
- m: für eine ganze Zahl von 10 bis 40, bevorzugt für eine ganze Zahl von 20 bis 35 und besonders bevorzugt für die Zahl 30, steht.

Bei einem besonders gut geeigneten nichtionischen Tensid dieses Typs handelt es sich um Ceteareth-30. Bei Ceteareth-30 handelt es sich um ein Gemisch aus Cetylalkohol und Stearylalkohol, die jeweils mit 30 Einheiten Ethylenoxid ethoxyliert sind. Das Gemisch aus Cetylalkohol und Stearylalkohol wird als Cetearylalkohol bezeichnet. Ceteareth-30 besitzt die CAS-Nummer 68439-49-6 und ist beispielsweise unter dem Handelsnamen Eumulgin B3 von der Firma BASF käuflich zu erwerben.

### weitere optionale Inhalttstoffe in den Mitteln (a) und/oder (b)

Zusätzlich zu den bereits beschriebenen erfindungswesentlichen Bestandteilen können die Mittel (a) und/oder (b) auch noch weitere optionale Inhaltsstoffe enthalten.

So können die Mittel (a) und/oder (b) beispielsweise ein filmbildendes Polymer enthalten. Das filmbildende Polymer kann zum Beispiel ausgewählt ist aus der Gruppe aus Polyvinylpyrrolidon (PVP), Vinylpyrrolidon/Vinylacetat-Copolymeren, Vinylpyrrolidon/Styren-Copolymeren, Vinylpyrrolidon/Ethylen-Copolymeren, Vinylpyrrolidon/Propylen-Copolymeren, Vinylpyrrolidon/Vinylcaprolactam-Copolymeren, Vinylpyrrolidon/Vinylformamid-Copolymeren und/oder Vinylpyrrolidon/Vinylalkohol-Copolymeren, explizit ganz besonders bevorzugt Polyvinylpyrrolidon (PVP).

Weitere geiegnete filmbildende Polymere können augewählt sein aus der Gruppe der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

Es haben sich insbesondere die filmbildenden Polymere als gut geeignet erwiesen, die aus der Gruppe der synthetischen Polymere, der durch radikalische Polymerisation erhältlichen Polymere oder der natürlichen Polymere ausgewählt werden.

Weitere besonders gut geeignete filmbildende Polymere können ausgewählt werden aus den Homopolymere oder Copolymeren von Olefinen, wie beispielsweise Cycloolefinen, Butadien, Isopren oder Styren, Vinylethern, Vinylamiden, den Estern oder Amiden von (Meth)Acrylsäure mit mindestens einer C₁-C₂₀-Alkylgruppe, einer Arylgruppe oder einer C2-C10-Hydroxyalkylgruppe.

Weitere filmbildende Polymere können ausgewählt sein aus den Homo- oder Copolymeren von Isooctyl-(meth)acrylat; Isononyl-(meth)acrylat; 2-Ethylhexyl-(meth)acrylat; Lauryl-(meth)acrylat); isopentyl-(meth)acrylat; n-Butyl-(meth)acrylat); Isobutyl-(meth)acrylat; Ethyl-(meth)acrylat; Methyl-(meth)acrylat; tert-Butyl-(meth)acrylat; Stearyl-(meth)acrylat; Hydroxyethyl-(meth)acrylat; 2-Hydroxypropyl-(methacrylat; 3-Hydroxypropyl-(meth)acrylat und/oder Gemischen hiervon.

Weitere filmbildende Polymere können ausgewählt sein aus den Homo- oder Copolymeren von (Meth)acrylamid; N-Alkyl-(meth)acrylamiden, insbesondere solchen mit C2-C18 Alkylgruppen, wie beispielsweise N-Ethyl-acrylamid, N-tert-butyl-acrylamid, le N-Octyl-crylamid; N-Di(C1-C4)alkyl-(meth)acrylamid.

Weitere bevorzugte anionische Copolymere sind beispielsweise Copolymere aus Acrylsäure, Methacrylsäure oder deren C₁-C₆-Alkylestern, wie sie unter der INCI-Deklaration Acrylates Copolymere vertrieben werden. Ein geeignetes Handelsprodukt ist beispielsweise Aculyn^{®} 33 der Firma Rohm & Haas. Weiterhin bevorzugt sind aber auch Copolymere aus Acrylsäure, Methacrylsäure oder deren C₁-C₆-Alkylestern und den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20.

Auf dem Markt befindliche ganz besonders bevorzugte Polymere sind beispielsweise Aculyn^{®} 22 (Acrylates/Steareth-20 Me-thacrylate Copolymer), Aculyn^{®} 28 (Acrylates/Beheneth-25 Methacrylate Copolymer), Structure 2001^{®} (Acryla-tes/Steareth-20 Itaconate Copolymer), Structure 3001^{®} (Acrylates/Ceteth-20 Itaconate Copolymer), Structure Plus^{®} (Acrylates/Aminoacrylates C10-30 Alkyl PEG-20 Itaconate Copolymer), Carbopol^{®} 1342, 1382, Ultrez 20, Ultrez 21 (Acrylates/C10-30 Alkyl Acrylate Crosspolymer), Synthalen W 2000^{®} (Acrylates/Palmeth-25 Acrylate Copolymer) oder das Rohme und Haas vertriebene Soltex OPT (Acrylates/C12-22 Alkyl methacrylate Copolymer).

Als geeignete Polymere auf der Basis von Vinyl-Monomeren können beispielsweise genannt werden die Homo- und Copolymere des N-Vinylpyrrolidons, des Vinylcaprolactams, des Vinyl-(C1-C6-)alkyl-Pyrrols, des Vinyl-oxazols, des Vinyl-thiazols, des Vinylpyrimidins, des Vinylimidazols.

Weiterhin ganz besonders gut geeignet sind die Copolymere Octylacrylamid/acrylates/ butylaminoethyl-methacrylate copolymer, wie es beispielsweise unter den Handelsnamen AMPHOMER^{®} ou LOVOCRYL^{®} 47 von NATIONAL STARCH kommerziell vertrieben wird, oder auch die Copolymere von Acrylates/Octylacrylamide die unter den Handelsnamen DERMACRYL^{®} LT und DERMACRYL^{®} 79 von NATIONAL STARCH vertrieben werden.

Als geeignete Polymere auf der Basis von Olefinen können beispielsweise genannt werden die Homo- und Copolymere des Ethylens, des Propylens, des Butens, des Isoprens und des Butadiens.

Im Rahmen einer weiteren Ausführungsform können als filmbildenden hydrophoben Polymere die Block-Copoylmere eingesetzt werden, die mindestens einen Block aus Styren oder den Derivaten des Styrens umfassen. Bei diesen Block-Copolymeren kann es sich um Copolymere handeln, die neben einem Styren-Block einen oder mehrere weitere Blöcke enthalten, wie beispielsweise Styren/Ethylen, Styren/Ethylen/Butylen, Styen/Butylen, Styren/Isopren, Styren/Butadien. Entsprechende Polymere werden von der BASF unter dem Handelsnamen "Luvitol HSB" kommerziell vertrieben.

In einer weiteren ganz besonders bevorzugten Ausführungsform ist ein erfindungsgemäßes Verfahren dadurch gekennzeichnet, dass das Mittel (a) und/oder (b) mindestens ein filmbildendes Polymer (b2) enthält, das ausgewählt ist aus der Gruppe aus Polyvinylpyrrolidon (PVP), Vinylpyrrolidon/Vinylacetat-Copolymeren, Vinylpyrrolidon/Styren-Copolymeren, Vinylpyrrolidon/Ethylen-Copolymeren, Vinylpyrrolidon/Propylen-Copolymeren, Vinylpyrrolidon/Vinylcaprolactam-Copolymeren, Vinylpyrrolidon/Vinylformamid-Copolymeren und/oder Vinylpyrrolidon/Vinylalkohol-Copolymeren, der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

Das oder die filmbildenden Polymere werden bevorzugt in bestimmten Mengenbereichen in den Mitteln (a) und/oder (b) eingesetzt. In diesem Zusammenhang hat es sich zur Lösung der erfindungsgemäßen Aufgabenstellung als besonders bevorzugt erwiesen, wenn das Mittel (b) - bezogen auf das Gesamtgewicht des Mittels (b) - ein oder mehrere Polymere in einer Gesamtmenge von 0,1 bis 25,0 Gew.-%, bevorzugt von 0,2 bis 20,0 Gew-%, weiter bevorzugt von 0,5 bis 15,0 Gew.-% und ganz besonders bevorzugt von 1,0 bis 7,0 Gew.-% enthält.

Die Mittel können zusätzlich ein oder mehrere Tenside enthalten. Unter dem Begriff Tenside werden grenzflächenaktive Substanzen verstanden. Man unterscheidet anionische Tenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wässriger Lösung stark hydratisiert sind.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ - Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Typische Beispiele für amphotere bzw. zwitterionische Tenside sind Alkylbetaine, Alkylamidobetaine, Amino-propionate, Aminoglycinate, Imidazoliniumbetaine und Sulfobetaine.

Besonders bevorzugte ampholytische Tenside sind das N-Kokosalkylaminopropionat, das Kokosacylaminoethylaminopropionat und das C₁₂ - C₁₈ - Acylsarcosin.

Weiterhin können die Mittel zusätzlich auch mindestens ein kationisches Tensid enthalten. Unter kationischen Tensiden werden Tenside, also grenzflächenaktive Verbindungen, mit jeweils einer oder mehreren positiven Ladungen verstanden. Kationische Tenside enthalten ausschließlich positive Ladungen. Üblicherweise sind diese Tenside aus einem hydrophoben Teil und einer hydrophilen Kopfgruppe aufgebaut, wobei der hydrophobe Teil in der Regel aus einem Kohlenwasserstoff-Gerüst (z.B. bestehend aus einer oder zwei linearen oder verzweigten Alkylketten) besteht, und die positive(n) Ladung(en) in der hydrophilen Kopfgruppe lokalisiert sind. Beispiele für kationische Tenside sind
- quartäre Ammoniumverbindungen, die als hydrophobe Reste ein oder zwei Alkylketten mit einer Kettenlänge von 8 bis 28 C-Atomen tragen können,
- quartäre Phosphoniumsalze, substituiert mit einer oder mehreren Alkylketten mit einer Kettenlänge von 8 bis 28 C-Atomen oder
- tertiäre Sulfonium-Salze.

Weiterhin kann die kationische Ladung auch in Form einer Onium-Struktur Bestandteil eines heterozyklischen Ringes (z.B. eines Imidazoliumringe oder einer Pyridiniumringes) sein. Neben der funktionellen Einheit, welche die kationische Ladung trägt, kann das kationische Tensid auch weitere ungeladene funktionelle Gruppen beinhalten, wie dies beispielsweise bei Esterquats der Fall ist. Die kationischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des jeweiligen Mittels - eingesetzt.

Weiterhin können die erfindungsgemäßen Mittel auch mindestens ein anionisches Tensid enthalten. Als anionische Tenside werden oberflächenaktive Mittel mit ausschließlich anionischen Ladungen (neutralisiert durch ein entsprechendes Gegenkation) bezeichnet. Beispiele für anionische Tenside sind Fettsäuren, Alkylsulfate, Alkylethersulfate und Ethercarbonsäuren mit 12 bis 20 C-Atomen in der Alkylgruppe und bis zu 16 Glykolethergruppen im Molekül.

Die anionischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des jeweiligen Mittels - eingesetzt.

Die Mittel können ferner auch noch weitere Wirk-, Hilfs- und Zusatzstoffe enthalten, wie beispielsweise Lösungsmittel, Fettbestandteile wie beispielsweise der C₈-C₃₀-Fettalkohole, der C₈-C₃₀-Fettsäuretriglyceride, der C₈-C₃₀-Fettsäuremonoglyceride, der C₈-C₃₀-Fettsäurediglyceride und/oder der Kohlenwasserstoffe; Polymere; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Zubereitungen bevorzugt in Mengen von jeweils 0,0001 bis 25 Gew.-%, insbesondere von 0,0005 bis 15 Gew.-%, bezogen auf das Gesamtgewicht des jeweiligen Mittels, eingesetzt.

Wie bereits zuvor beschrieben, besteht das Mittel (a) jedoch besonders bevorzugt im wesentlichen aus den Inhaltstoffen (a1), (a2) sowie gegebenenfalls (a3). Falls das Mittel (a) daher einen der zuvor beschriebenen weiteren optionalen Inhaltsstoffe enthalten sollte, so werden diese besonders bevorzugt im Mittel (a) nur in sehr geringen Mengen eingesetzt.

### Herstellung der Anwendungsmischung durch Vermischen der Mittel (a) und (b)

In Schritt (3) des erfindungsgemäßen Verfahrens wird durch Vermischen der Mittel (a) und (b) eine anwendungsbereite Mischung hergestellt. Anders ausgedrückt wird in diesem Verfahrensschritt der Premix oder das Konzentrat (a), d.h. die bevorzugt wasserarme, hochkonzentrierte Abmischung aus Aminosilikon (a1), farbgebender Verbindung (a2) und optional Lösungsmittel (a3), mit einer kosmetischen Trägerformulierung (b) mit geringem Wassergehalt (b1) und Fettbestandteil (b2) vermischt.

Prinzipiell können verschiedene Mengen des Mittels (a) mit dem Mittel (b) vermischt werden, so sind prinzipell Mischungsverhältniss (a)/(b) von 1:200 bis 200:1 denkbar.

Da der Premix (a) jedoch bevorzugt ein Konzentrat darstellt, hat es sich als ganz besonders bevorzugt erwiesen, das Mittel (a) in geringen Mengen einzusetzen und diese mit im Vergleich höheren Mengen des Mittels (b) zu verdünnen.

Besonders bevorzugt ist es, wenn die Anwendungsmischung durch Vermischen der Mittel (a) und (b) im Mengenverhältnis (a)/(b) von1:2 bis 1:200, bevorzugt von 1:5 bis 1:100, weiter bevorzugt von 1:5 bis 1:40 und ganz besonders bevorzugt von 1:15 bis 1:20 hergestellt wird.

Bei einem Mengenverhältnis (a)/(b) von 1:5 können beispielsweise 10 g Mittel (a) mit 50 g Mittel (b) vermischt werden.

Bei einem Mengenverhältnis (a)/(b) von 1:100 können beispielsweise 2 g Mittel (a) mit 200 g Mittel (b) vermischt werden.

Bei einem Mengenverhältnis (a)/(b) von 1:15 können beispielsweise 15 g Mittel (a) mit 225 g Mittel (b) vermischt werden.

Bei einem Mengenverhältnis (a)/(b) von 1:25 können beispielsweise 4 g Mittel (a) mit 100 g Mittel (b) vermischt werden.

Im Rahmen einer weiteren bevorzugten Ausführungsform ist ein erfindungsgemäßes Verfahren gekennzeichnet durch die
(3) Herstellung einer Anwendungsmischung durch Vermischen der Mittel (a) und (b) im Mengenverhältnis (a)/(b) von 1:2 bis 1:200, bevorzugt von 1:5 bis 1:100, weiter bevorzugt von 1:5 bis 1:40 und ganz besonders bevorzugt von 1:15 bis 1:20.

Die pH-Werte der Mittel (a) und (b) werden bevorzugt so eingestellt, dass auch die aus (a) und (b) hergestellte Anwendungsmischung einen neutral bis alkalischen pH-Wert besitzt. Ganz besonders bevorzugt besitzt die Anwendungsmischung einen alkalischen pH-Wert im Bereich von 7,0 bis 11,5 bevorzugt von 8,0 bis 11,0, und besonders bevorzugt von 8,5 bis 10,5. Unter basischen Bedingungen kann das aminofunktionalisierte Silikonpolymer (a1) besonders gut und ohne Protonierung gelöst bzw. dispergiert werden.

Im Rahmen einer weiteren bevorzugten Ausführungsform ist ein erfindungsgemäßes Verfahren dadurch gekennzeichnet, dass die durch Vermischen der Mittel (a) und (b) hergestellte Anwendungsmischung einen pH-Wert von 7,0 bis 11,5 bevorzugt von 8,0 bis 11,0, und besonders bevorzugt von 8,5 bis 10,5 besitzt.

Zur Einstellung des gewünschten pH-Wertes kann das Mittel (a) und/oder (b) mindestens ein Alkalisierungsmittel enthalten. Bei den pH-Werten im Sinne der vorliegenden Erfindung handelt es sich um pH-Werte, die bei einer Temperatur von 22°C gemessen wurden.

Als Alkalisierungsmittel können die Mittel beispielsweise Ammoniak, Alkanolamine und/oder basische Aminosäuren enthalten.

Die in dem erfindungsgemäßen Mittel einsetzbaren Alkanolamine werden bevorzugt ausgewählt aus primären Aminen mit einem C₂-C₆-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol.

Erfindungsgemäß besonders bevorzugte Alkanolamine werden ausgewählt aus 2-Aminoethan-1-ol und/oder 2-Amino-2-methylpropan-1-ol. Eine besonders bevorzugte Ausführungsform ist daher dadurch gekennzeichnet, dass das erfindungsgemäße Mittel als Alkalisierungsmittel ein Alkanolamin ausgewählt aus 2-Aminoethan-1-ol und/oder 2-Amino-2-methylpropan-1-ol enthält.

Als Aminosäure im Sinne der Erfindung gilt eine organische Verbindung, die in ihrer Struktur mindestens eine protonierbare Aminogruppe und mindestens eine -COOH- oder eine -SO₃H-Gruppe enthält. Bevorzugte Aminosäuren sind Aminocarbonsäuren, insbesondere α-(alpha)-Aminocarbonsäuren und ω-Aminocarbonsäuren, wobei α-Aminocarbonsäuren besonders bevorzugt sind.

Unter basischen Aminosäuren sind erfindungsgemäß solche Aminosäuren zu verstehen, welche einen isoelektrischen Punkt pl von größer 7,0 besitzen.

Basische α-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist ein erfindungsgemäßes Mittel daher dadurch gekennzeichnet, dass es sich bei dem Alkalisierungsmittel um eine basische Aminosäure aus der Gruppe Arginin, Lysin, Ornithin und/oder Histidin handelt.

Darüber hinaus kann das Mittel weitere Alkalisierungsmittel, insbesondere anorganische Alkalisierungsmittel enthalten. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Ganz besonders bevorzugte Alkalisierungsmittel sind Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

In einer weiteren ganz besonders bevorzugten Ausführungsform ist ein erfindungsgemäßes Verfahren dadurch gekennzeichnet, dass das Färbemittel (a) mindestens ein Alkalisierungsmittel aus der Gruppe aus Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methyl-propan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat enthält.

### Applizieren der Anwendungsmischung

In Schritt (4) des erfindungsgemäßen Verfahrens wird die in Schritt (3) hergestellte Anwendungsmischung auf das keratinische Material, bei dem es sich ganz besonders bevorzugt um menschliche Haare handelt, appliziert.

Bevorzugt wird die Anwendungsmischung innerhalb eines Zeitraums von 1 bis 120 Minuten, bevorzugt von 1 bis 60 Minuten, weiter bevorzugt 1 bis 30 Minuten und ganz besonders bevorzugt von 1 bis 15 Minuten nach ihrer Herstellung in Schritt (3) auf das Keratinmaterial (bzw. auf die Haare) appliziert.

In einer weiteren bevorzugten Ausführungsform ist ein erfindungsgemäßes Verfahren gekennzeichnet durch das
(4) Applizieren Anwendungsmischung auf dem keratinischen Material innerhalb eines Zeitraums von 1 bis 120 Minuten, bevorzugt von 1 bis 60 Minuten, weiter bevorzugt 1 bis 30 Minuten und ganz besonders bevorzugt von 1 bis 15 Minuten nach ihrer Herstellung in Schritt (3).

### Einwirken der Anwendungsmischung auf das Keratinmaterial

In Schritt (5) des erfindungsgemäßen Verfahrens wird die Anwendungsmischung nach ihrer Applikation auf das keratinische Material einwirken gelassen. In diesem Zusammenhang sind verschiedene Einwirkzeiten von beispielsweise 30 Sekunden bis 60 Minuten denkbar.

Ein großer Vorteil des erfindungsgemäßen Färbesystems liegt jedoch darin, dass auch in sehr kurzen Zeiträumen nach kurzen Einwirkzeiten ein intensive Farbergebnis erzielt werden kann. Aus diesem Grund ist es von Vorteil, wenn die Anwendungsmischung nach ihrer Applikation nur für vergleichsweise kurze Zeiträume von 30 Sekunden bis 15 Minuten, bevorzugt von 30 Sekunden bis 10 Minuten, und besonders bevorzugt von 1 bis 5 Minuten auf dem Keratinmaterial verbleibt.

In einer weiteren bevorzugten Ausführungsform ist ein erfindungsgemäßes Verfahren gekennzeichnet durch das
(5) Einwirken der in Schritt (4) applizierten Anwendungsmischung auf dem keratinischen Material für einen Zeitraum von 30 Sekunden bis 15 Minuten, bevorzugt von 30 Sekunden bis 10 Minuten, und besonders bevorzugt von 1 bis 5 Minuten

### Ausspülen der Anwendungsmischung

Im Anschluss an das Einwirken der Anwendungsmischung auf das Keratinmaterial wird diese schließlich in Schritt (6) mit Wasser ausgespült.

Hierbei kann die Awendungsmischung in einer Ausführungsform nur mit Wasser, d.h. ohne Zuhilfenahme eines Nachbehandlungsmittels oder eines Shampoos, ausgewaschen werden. Auch die Anwendung eines Nachbehandlungsmittels oder Conditioners in Schritt (6) ist prinzipiell denkbar.

Zur Lösung der erfindungsgemäßen Aufgabenstellung und zur Erhöhung des Anwendungskomforts hat es sich jedoch als ganz besonders bevorzugt herausgestellt, das Ausspülen der Anwendungsmischung in Schritt (6) ausschließlich mit Wasser ohne Zuhilfenahme eines weiteren Nachbehandlungsmittels, Shampoos oder Conditioners vorzunehmen.

In einer weiteren bevorzugten Ausführungsform ist ein erfindungsgemäßes Verfahren gekennzeichnet durch das
(6) Ausspülen der Anwendungsmischung ausschließlich mit Wasser.

### Abfolge der Verfahrensschritte

Das erfindungsgemäße Verfahren umfasst die Schritte (1) bis (6).

In Schritt (1) wird das Mittel (a) bereitgestellt, Schritt (2) umfasst die Bereitstellung des Mittels (b). Diese beiden Schritte müssen zeitlich nicht zwingend nacheinander erfolgen, sondern können auch gleichzeitig ablaufen.

So kann Schritt (1) vor Schritt (2) erfolgen, die Schritte (1) und (2) können gleichzeitig ablaufen oder aber Schritt (2) erfolgt vor Schritt (1).

Werden dem Anwender beispielsweise die Mittel (a) und (b) in einer Mehrkomponenten-Verpackungseinheit zur Vergügung gestellt, so werden beide Mittel gleichzeitig bereitgestellt, und es bleibt dem Anwender überlassen, welches Mittel er zuerst aus der Verpackung entnimmt.

Die Herstellung einer Anwendungsmischung durch Vermischen der Mittel (a) und (b) in Schritt (3) kann nur nach Bereitstellung der beiden Mittel (a) und (b) erfolgen.

Das Applizieren der Anwendungsmischung in Schritt (4) kann erst nach deren Herstellung in Schritt (3) erfolgen.

Analog kann das Einwirken der Anwendungsmischung in Schritt (5) erst nach deren Aufbringen auf das Keratinmaterial ergolgen, und das Ausspülen der Anwendungmischung in Schritt (6) erfolgt nach deren Einwirken in Schritt (5).

### Mehrkomponenten-Verpackungseinheit

Zur Erhöhung des Anwenderkomforts werden dem Anwender bevorzugt alle benötigten Mittel in Form einer Mehrkomponenten-Verpackungseinheit (Kit-of-parts) zur Verfügung gestellt.

Ein zweiter Gegenstand der vorliegenden Erfindung ist daher eine Mehrkomponenten-Verpackungseinheit (Kit-of-parts) zum Färben von keratinischem Material, insbesondere menschlichen Haaren, umfassend getrennt voneinander konfektioniert
- einen ersten Container mit einem Mittel (a), wobei das Mittel (a) enthält:
   (a1) mindestens ein aminofunktionalisiertes Silikonpolymer, und
   (a2) mindestens eine farbgebende Verbindung, und
   (a3) gegebenenfals mindestens ein Lösungsmittel (a3), und
- einen zweiten Container mit einem Mittel (b), wobei das Mittel (b) - bezogen auf das Gesamtgewicht des Mittels (b) - enthält:
   (b1) 0 bis 50 Gew.-% Wasser, und
   (b2) mindestens einen Fettbestandteil, und
   (b3) gegebenenfalls mindestens ein nichtionisches Tensid.
wobei die Inhaltsstoffe (a1), (a2), (a3), (b1), (b2) und (b3) bereits bei der Beschreibung des ersten Erfindungsgegenstands im Detail offenbart wurden.

Die im Mittel (a) des Kits enthaltenen aminofunktionalisierten Silikonpolymere (a1) entsprechen den aminofunktionalisierten Silikonpolymere (a1), die auch im Mittel (a) des zuvor beschriebenen Verfahrens eingesetzt wurden.

Die im Mittel (a) des Kits enthaltenen farbgebenden Verbindungen (a2) entsprechen den farbgebenden Verbindungen (a2), die auch im Mittel (a) des zuvor beschriebenen Verfahrens eingesetzt wurden.

Die im Mittel (a) des Kits gegebenenfalls enthaltenen Lösungsmittel (a3) entsprechen den Lösungsmitteln (a3), die auch im Mittel (a) des zuvor beschriebenen Verfahrens eingesetzt werden können.

Die im Mittel (b) des Kits enthaltenen Fettbestandteile (b2),entsprechen den Fettbestandteilen (b2), die auch im Mittel (a) des zuvor beschriebenen Verfahrens eingesetzt wurden.

Die im Mittel (b) des Kits optional enthaltenen nichtionischen Tenside (b3),entsprechen den nichtionischen Tensiden (b3), die auch im Mittel (a) des zuvor beschriebenen Verfahrens eingesetzt wurden.

### Anwendungsbereites Mittel

Durch Vermischen der beiden Mittel (a) und (b) wird ein anwendungsbereites Färbemittel hergestellt, mit dem sich das Keratinmaterial intensiv und homogen färben lässt.

Wird wie zuvor im Rahmen einer ganz besonders bevorzugten Auführungsform beschrieben ein wasserfreies Mittel (a) zum Vermischen mit dem Mittel (b) eingesetzt, so enthält das anwendungsbereite Mittel
(a1) mindestens ein aminofunktionalisiertes Silikonpolymer, und
(a2) mindestens eine farbgebende Verbindung, und
(a3) gegebenenfals mindestens ein Lösungsmittel (a3), und
(b1) 0 bis 50 Gew.-% Wasser, und
(b2) mindestens einen Fettbestandteil, und
(b3) gegebenenfalls mindestens ein nichtionisches Tensid,
wobei die Inhaltsstoffe (a1), (a2), (a3), (b1), (b2) und (b3) bereits bei der Beschreibung des ersten Erfindungsgegenstands im Detail offenbart wurden.

Betreffend die weiteren bevorzugten Ausführungsformen der erfindungsgemäßen Mehrkomponenten-Verpackungseinheit und des anwendungsbereiten Mittels gilt *mutatis mutantis* das zum erfindungsgemäßen Verfahren gesagte.

### Beispiele

### 1. Formulierungen

Es wurden die folgenden Formulierungen hergestellt:

| Mittel (a) (Premix) | Mittel (a) |
|---|---|
| Lavanya Zuni (organisches Pigment,Neelikon Red, 111P0200, CI 12490) | 1,0 g |
| Dow Corning 2-8566 (Siloxanes and Silicones, 3-[(2-Aminoethyl)amino]-2-methylpropyl Me, Di-Me-Siloxane" | 1,0 g |
| 1,2-Propandiol | 10,0 g |
| Gesamtmenge Premix (a) | 12,0 g |

| Mittel (b) Träger-Formulierung | (b1) | (b2) | (b3) |
|---|---|---|---|
| Cutina GMS V (INCI: Glyceryl stearate, Glyceol Mono/dipalmitate/stearate) CAS-Nr. 85251-77-0 | 13,0 g | 13,0 g | 13,0 g |
| Dehydol LS 2 deo N (Fatty alcohols C12-14, ethoxylated (2 EO) | 4,5 g | 4,5 g | 4,5 g |
| 1,2-Propandiol | 6,0 g | 6,0 g | 6,0 g |
| Kaliumhydroxid, 50 %ige wässrige Lösung | 0,05 g | 0,05 g | 0,05 g |
| Phenoxyethanol | 0,5 g | 0,5 g | 0,5 g |
| Natriumsalicylat | 0,5 g | 0,5 g | 0,5 g |
| Wasser | --- | 10 g | 20 g |
| Cetiol 868 (Ethylhexyl stearate, CAS-Nr. 91031-48-0) | ad 100 g | ad 100 g | ad 100 g |
| Gesamtmenge Träger-Basis (b) | 100 g | 100 g | 100 g |

| Mittel (b) Träger-Formulierung | (b4) | (b5) | (b6) |
|---|---|---|---|
| Cutina GMS V (INCI: Glyceryl stearate, Glyceol Mono/dipalmitate/stearate) CAS-Nr. 85251-77-0 | 13,0 g | 13,0 g | 13,0 g |
| Dehydol LS 2 deo N (Fatty alcohols C12-14, ethoxylated (2 EO) | 4,5 g | 4,5 g | 4,5 g |
| 1,2-Propandiol | 6,0 g | 6,0 g | 6,0 g |
| Kaliumhydroxid, 50 %ige wässrige Lösung | 0,05 g | 0,05 g | 0,05 g |
| Phenoxyethanol | 0,5 g | 0,5 g | 0,5 g |
| Natriumsalicylat | 0,5 g | 0,5 g | 0,5 g |
| Wasser | 30,0 g | 50,0 g | 70,0 g |
| Cetiol 868 (Ethylhexyl stearate, CAS-Nr. 91031-48-0) | ad 100 g | ad 100 g | ad 100 g |
| Gesamtmenge Träger-Basis (b) | 100 g | 100 g | 100 g |

### 2. Anwendung

Zur Herstellung der Anwendungsmischung wurden jeweils 12 g des Mittels (a) mit 100 g des jeweiligen Mittels (b) vermischt. Jede Anwendungsmischung wurde auf Haarsträhnen (Kerling, Euronaturhaar weiß, Flottenverhältnis: 1 g Anwendungsmischung pro g Haarsträhne) appliziert. Die Anwendungsmischung wurde für drei Minuten einwirken gelassen. Im Anschluss daran wurden die Haarsträhne gründlich (1 Minute) mit Wasser ausgewaschen, getrocknet und dann visuell unter der Tageslichtlampe bewertet. Das Griffgefühl und die Farbintensität jeder gefärbten Strähne wurde durch geschulte Personen bewertet.

| Anwendungsmischung | AWM 1 | AWM 2 | AWM 3 |
|---|---|---|---|
| Mittel (a) + Mittel (b) | (a) + (b1) | (a) + (b2) | (a) + (b3) |
| Farbintensität | rot mittel | rot mittel | rot gut |
| Griffgefühl | sehr gut | sehr gut | sehr gut |
| Wasserghalt im Mittel (b) | 0 Gew.-% | 10 Gew.-% | 20 Gew.-% |

| Anwendungsmischung | AWM 4 | AWM 5 | AWM 6 |
|---|---|---|---|
| Mittel (a) + Mittel (b) | (a) + (b4) | (a) + (b5) | (a) + (b6) |
| Farbintensität | rot sehr gut | rot sehr gut | rot sehr gut |
| Griffgefühl | gut | mittel | schlecht |
| Wasserghalt im Mittel (b) | 30 Gew.-% | 50 Gew.-% | 70 Gew.-% |

Mit den Anwendungsmischungen AWM 3 und AMW 4 konnten Ergebnisse erhalten werden, die sowohl im Hinblick auf die Farbintensität als auch im Hinblick auf das Griffgefühl der Haare gut bis sehr gut waren.

## Patentansprüche

1. Verfahren zum Färben von keratinischem Material, insbesondere menschlichen Haaren, umfassend die folgenden Schritte:
(1) Bereitstellung eines Mittels (a), wobei das Mittel (a) - bezogen auf das Gesamtgewicht des Mittels (a) - 0 bis 10 Gew.-% Wasser und
(a1) mindestens ein aminofunktionalisiertes Silikonpolymer, und
(a2) mindestens eine farbgebende Verbindung enthält, und
(2) Bereitstellung eines Mittels (b), wobei das Mittel (b) - bezogen auf das Gesamtgewicht des Mittels (b) - enthält:
(b1) 0 bis 50 Gew.-% Wasser und
(b2) mindestens einen Fettbestandteil,
(3) Herstellung einer Anwendungsmischung durch Vermischen der Mittel (a) und (b),
(4) Applizieren der in Schritt (3) hergestellten Anwendungsmischung auf dem keratinischen Material,
(5) Einwirken der in Schritt (4) applizierten Anwendungsmischung auf dem keratinischen Material und
(6) Ausspülen der Anwendungsmischung mit Wasser.

2. Verfahren nach Anspruch 1, **dadurch gekennzeichnet, dass** das Mittel (a) mindestens ein aminofunktionalisiertes Silikonpolymer (a1) mit mindestens einer sekundären Aminogruppe enthält.

3. Verfahren nach einem der Ansprüche 1 bis 2 **dadurch gekennzeichnet, dass** das Mittel (a) mindestens ein aminofunktionalisiertes Silikonpolymer (a1) enthält, das mindestens eine Struktureinheit der Formel (Si-Amino) umfasst, 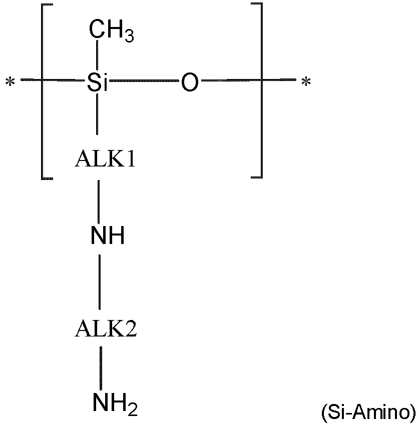 wobei
ALK1 und ALK2 unabhängig voneinander für eine lineare oder verzweigte, zweiwertige C₁-C₂₀-Alkylengruppe stehen.

4. Verfahren nach einem der Ansprüche 1 bis 3, **dadurch gekennzeichnet, dass** das Mittel (a) mindestens ein aminofunktionalisiertes Silikonpolymer (a1) enthält, das Struktureinheiten der Formel (Si-I) und der Formel (Si-II) umfasst

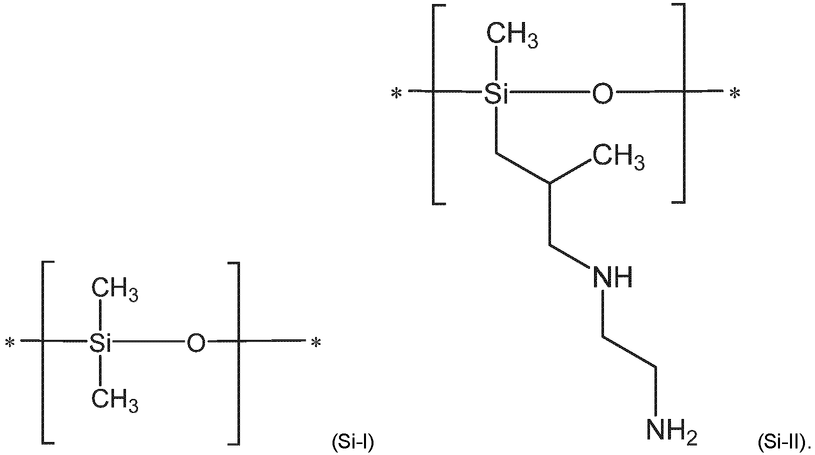

5. Verfahren nach einem der Ansprüche 1 bis 4, **dadurch gekennzeichnet, dass** das Mittel (a) - bezogen auf das Gesamtgewicht des Mittels (a) - ein oder mehrere aminofunktionalisierte Silikonpolymere (a1) in einer Gesamtmenge von 2,0 bis 95,0 Gew.-%, bevorzugt 4,0 bis 70,0 Gew.-%, weiter bevorzugt von 6,0 bis 50,0 Gew.-% und ganz besonders bevorzugt von 8,0 bis 20,0 Gew.-% enthält.

6. Verfahren nach einem der Ansprüche 1 bis 5, **dadurch gekennzeichnet, dass** das Mittel (a) mindestens eine farbgebende Verbindung (a2) aus der Gruppe der Pigmente, der direktziehenden Farbstoffe, der photochromen Farbstoffe und der thermochromen Farbstoffe enthält.

7. Verfahren nach einem der Ansprüche 1 bis 6, **dadurch gekennzeichnet, dass** das Mittel (a) mindestens eine farbgebende Verbindung (a2) aus der Gruppe der anorganischen Pigmente enthält, die bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

8. Verfahren nach einem der Ansprüche 1 bis 7, **dadurch gekennzeichnet, dass** das Mittel (a) mindestens eine farbgebende Verbindung (a2) aus der Gruppe der organischen Pigmente enthält, die bevorzugt ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern Cl 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

9. Verfahren nach einem der Ansprüche 1 bis 8, **dadurch gekennzeichnet, dass** das Mittel (a) - bezogen auf das Gesamtgewicht des Mittels (a) - ein odere mehrere Pigmente in einer Gesamtmenge von 2,0 bis 95,0 Gew.-%, bevorzugt 4,0 bis 70,0 Gew.-%, weiter bevorzugt von 6,0 bis 50,0 Gew.-% und ganz besonders bevorzugt von 8,0 bis 30,0 Gew.-% enthält.

10. Verfahren nach einem der Ansrpüche 1 bis 9, **dadurch gekennzeichnet, dass** das Mittel (a) mindestens ein Lösungsmittel (a3) aus der Gruppe aus 1,2-Propylenglycol, 1,3-Propylenglycol, Ethylenglycol, 1,2- Butylenglycol, Dipropylenglycol, Ethanol, Isopropanol, Diethylenglycolmonoethylether, Glycerin, Phenoxyethanol und Benzylalkohol, ganz besonders bevorzugt 1,2-Propylenglycol, enthält.

11. Verfahren nach einem der Ansprüche 1 bis 10, **dadurch gekennzeichnet, dass** das Mittel (a) - bezogen auf das Gesamtgewicht des Mittels (a) - ein oder mehrere Lösungsmittel (a3) in einer Gesamtmenge von 1,0 bis 95,0 Gew.-%, bevorzugt 20,0 bis 90,0 Gew.-%, weiter bevorzugt 40,0 bis 85,0 Gew.-% und ganz besonders bevorzugt 60,0 bis 85,0 Gew.-% enthält.

12. Verfahren nach einem der Ansprüche 1 bis 11, **dadurch gekennzeichnet, dass** das Mittel (a) - bezogen auf das Gesamtgewicht des Mittels (a) - 0 bis 5 Gew.-%, bevorzugt weniger als 0 bis 3 Gew.-%, und ganz besonders bevorzugt weniger als 1,0 Gew.-% Wasser enthält.

13. Verfahren nach einem der Ansprüche 1 bis 12, **dadurch gekennzeichnet, dass** die Bestandteile (a1), (a2) und (a3) - bezogen auf das Gesamtgewicht des Mittels (a) - zusammen einen Gewichtsanteil von mindestens 70,0 Gew.-%, bevorzugt von mindestens 80,0 Gew.-%, weiter bevorzugt von mindestens 90,0 Gew.-% und ganz besonders bevorzugt von mindestens 98,0 Gew.-% besitzen.

14. Verfahren nach einem der Ansprüche 1 bis 13, **dadurch gekennzeichnet, dass** das Mittel (b) - bezogen auf das Gesamtgewicht des Mittels (b) - 0 bis 45 Gew.-%, bevorzugt 5 bis 40 Gew.-%, weiter bevorzugt 10 bis 35 Gew.-% und ganz besonders bevorzugt 15 bis 35 Gew.-% Wasser (b1) enthält

15. Verfahren nach einem der Ansprüche 1 bis 14, **dadurch gekennzeichnet, dass** das Mittel (b) einen oder mehrere Fettbestandteile (b2) aus der Gruppe der Esteröle, der C₁₂-C₃₀-Fettalkohole, der C₁₂-C₃₀-Fettsäuretriglyceride, der C₁₂-C₃₀-Fettsäuremonoglyceride, der C₁₂-C₃₀-Fettsäure-diglyceride und/oder der Kohlenwasserstoffe enthält.

16. Verfahren nach einem der Ansprüche 1 bis 15, **dadurch gekennzeichnet, dass** das Mittel (b) mindestens einen Fettbestandteil (b2) aus der Gruppe der Ester einer C₆-C₃₀-Alkancarbonsäure mit einem aliphatischen C₂-C₃₀-Monoalkohol enthält.

17. Verfahren nach einem der Ansprüche 1 bis 16, **dadurch gekennzeichnet, dass** das Mittel (b) mindestens einen Fettbestandteil (b2) aus der Gruppe aus Stearinsäure-2-ethylhexylester, Isopropylmyristat, Isononansäure-C16-18-alkylester, 2-Ethylhexylpalmitat, Cetyloleat, Kokosfettalkoholcaprinat, Kokosfettalkoholcaprylat, n-Butylstearat, Oleylerucat, Isopropylpalmitat, Oleyloleat, Laurinsäurehexylester, Myristylmyristat, Cetearyl Isononanoate und Ölsäuredecylester enthält.

18. Verfahren nach einem der Ansprüche 1 bis 17, **dadurch gekennzeichnet, dass** das Mittel (b) - bezogen auf das Gesamtgewicht des Mittels (b) - einen oder mehrere Fettbestandteile in einer Gesamtmenge von 30 bis 99 Gew.-%, bevorzugt von 40 bis 97 Gew.-%, weiter bevorzugt von 50 bis 95 Gew.-% und ganz besonders bevorzugt von 60 bis 90 Gew.-% enthält.

19. Verfahren nach einem der Ansprüche 1 bis 18, **dadurch gekennzeichnet, dass** das Mittel (b) mindestens ein nichtionisches Tensid (b3) enthält.

20. Verfahren nach einem der Ansprüche 1 bis 19, **gekennzeichnet durch** die
(3) Herstellung einer Anwendungsmischung durch Vermischen der Mittel (a) und (b) im Mengenverhältnis (a)/(b) von 1:2 bis 1:200, bevorzugt von 1:5 bis 1:100, weiter bevorzugt von 1:5 bis 1:40 und ganz besonders bevorzugt von 1:15 bis 1:20.

21. Verfahren nach einem der Ansprüche 1 bis 20, **gekennzeichnet durch** das
(4) Applizieren der Anwendungsmischung auf dem keratinischen Material innerhalb eines Zeitraums von 1 bis 120 Minuten, bevorzugt von 1 bis 60 Minuten, weiter bevorzugt 1 bis 30 Minuten und ganz besonders bevorzugt von 1 bis 15 Minuten nach ihrer Herstellung in Schritt (3).

22. Verfahren nach einem der Ansprüche 1 bis 21, **gekennzeichnet durch** das
(5) Einwirken der in Schritt (4) applizierten Anwendungsmischung auf dem keratinischen Material für einen Zeitraum von 30 Sekunden bis 15 Minuten, bevorzugt von 30 Sekunden bis 10 Minuten, und besonders bevorzugt von 1 bis 5 Minuten.

23. Mehrkomponenten-Verpackungseinheit (Kit-of-parts) zum Färben von keratinischem Material, insbesondere menschlichen Haaren, umfassend getrennt voneinander konfektioniert
- einen ersten Container mit einem Mittel (a), wobei das Mittel (a) - bezogen auf das Gesamtgewicht des Mittels (a) - 0 bis 10 Gew.-% Wasser, und
(a1) mindestens ein aminofunktionalisiertes Silikonpolymer, und
(a2) mindestens eine farbgebende Verbindung, und
(a3) gegebenenfals mindestens ein Lösungsmittel (a3) enthält, und
- einen zweiten Container mit einem Mittel (b), wobei das Mittel (b) - bezogen auf das Gesamtgewicht des Mittels (b) - enthält:
(b1) 0 bis 50 Gew.-% Wasser, und
(b2) mindestens einen Fettbestandteil, und
(b3) gegebenenfalls mindestens ein nichtionisches Tensid.
wobei die Inhaltsstoffe (a1), (a2), (a3), (b1), (b2) und (b3) in einem der Ansprüche 1 bis 19 definiert sind.

## Claims

1. Method for dyeing keratinous material, in particular human hair, comprising the following steps:
(1) providing an agent (a), wherein the agent (a) comprises, based on the total weight of the agent (a), 0 to 10 wt% water and
(a1) at least one amino-functionalized silicone polymer, and
(a2) at least one coloring compound, and
(2) Provision of an agent (b), wherein the agent (b) contains, based on the total weight of the agent (b):
(b1) 0 to 50 wt% water and
(b2) at least one fatty component,
(3) Preparation of an application mixture by mixing the agents (a) and (b),
(4) applying the application mixture prepared in step (3) to the keratinous material,
(5) Allowing the application mixture applied in step (4) to act on the keratinous material, and
(6) Rinsing off the application mixture with water.

2. Method according to claim 1, **characterized in that** the agent (a) contains at least one amino-functionalized silicone polymer (a1) with at least one secondary amino group.

3. Method according to one of claims 1 to 2, **characterized in that** the agent (a) contains at least one amino-functionalized silicone polymer (a1) comprising at least one structural unit of the formula (Si-amino) 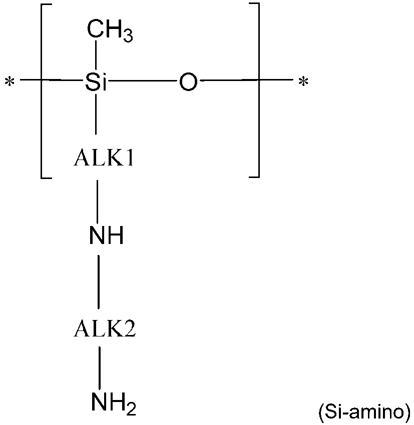 where
ALK1 and ALK2 independently represent a linear or branched, divalent C₁-C20-alkylene group.

4. Method according to one of claims 1 to 3, **characterized in that** the agent (a) contains at least one amino-functionalized silicone polymer (a1) comprising structural units of formula (Si-I) and formula (Si-II)

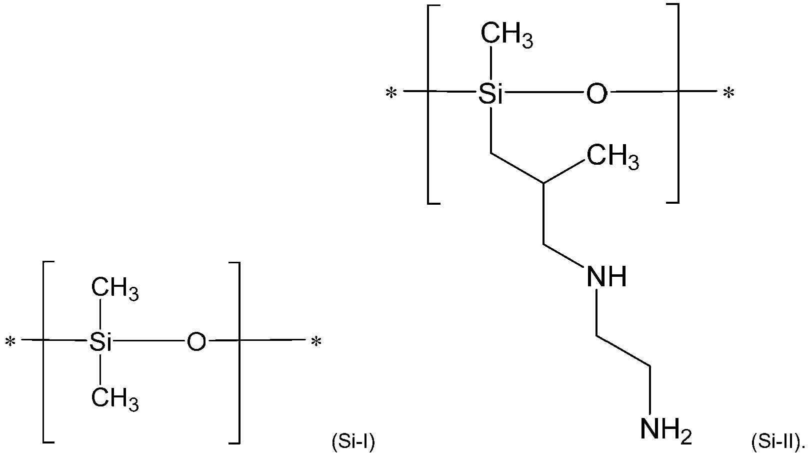

5. Method according to one of claims 1 to 4, **characterized in that** the agent (a) contains, based on the total weight of the agent (a), one or more amino-functionalized silicone polymers (a1) in a total amount of 2.0 to 95.0 wt.%, preferably 4.0 to 70.0 wt.%, more preferably from 6.0 to 50.0 wt.%, and most preferably from 8.0 to 20.0 wt.%.

6. Method according to one of claims 1 to 5, **characterized in that** the agent (a) contains at least one coloring compound (a2) from the group of pigments, direct dyes, photochromic dyes, and thermochromic dyes.

7. Method according to one of claims 1 to 6, **characterized in that** the agent (a) contains at least one coloring compound (a2) from the group of inorganic pigments, which is preferably selected from the group of colored metal oxides, metal hydroxides, metal oxide hydrates, silicates, metal sulfides, complex metal cyanides, metal sulfates, bronze pigments, and/or colored pigments based on mica or glimmer, which are coated with at least one metal oxide and/or one metal oxychloride.

8. Method according to one of claims 1 to 7, **characterized in that** the agent (a) contains at least one coloring compound (a2) from the group of organic pigments, which is preferably selected from the group consisting of carmine, quinacridone, phthalocyanine, sorghum, blue pigments with the Color Index numbers CI 42090, CI 69800, Cl 69825, CI 73000, Cl 74100, CI 74160, yellow pigments with the Color Index numbers Cl 11680, Cl 11710, Cl 15985, Cl 19140, CI 20040, CI 21100, CI 21108, CI 47000, Cl 47005, green pigments with Color Index numbers Cl 61565, Cl 61570, Cl 74260, orange pigments with Color Index numbers CI 11725, Cl 15510, CI 45370, Cl 71105, red pigments with Color Index numbers CI 12085, Cl 12120, Cl 12370, Cl 12420, CI 12490, CI 14700, Cl 15525, CI 15580, CI 15620, Cl 15630, Cl 15800, Cl 15850, CI 15865, CI 15880, CI 17200, CI 26100, Cl 45380, Cl 45410, CI 58000, CI 73360, Cl 73915, and/or Cl 75470.

9. Method according to one of claims 1 to 8, **characterized in that** the agent (a) contains, based on the total weight of the agent (a), one or more pigments in a total amount of 2.0 to 95.0 wt.%, preferably 4.0 to 70.0 wt.%, more preferably from 6.0 to 50.0 wt.% and most preferably from 8.0 to 30.0 wt.%.

10. Method according to one of claims 1 to 9, **characterized in that** the agent (a) contains at least one solvent (a3) from the group consisting of 1,2-propylene glycol, 1,3-propylene glycol, ethylene glycol, 1,2-butylene glycol, dipropylene glycol, ethanol, isopropanol, diethylene glycol monoethyl ether, glycerin, phenoxyethanol, and benzyl alcohol, with 1,2-propylene glycol being particularly preferred.

11. Method according to one of claims 1 to 10, **characterized in that** the agent (a)
- based on the total weight of the agent (a) - contains one or more solvents (a3) in a total amount of 1.0 to 95.0 wt.%, preferably 20.0 to 90.0 wt.%, more preferably 40.0 to 85.0 wt.%, and most preferably 60.0 to 85.0 wt.%.

12. Method according to one of claims 1 to 11, **characterized in that** the agent (a) - based on the total weight of the agent (a) - contains 0 to 5 wt.%, preferably less than 0 to 3 wt.%, and most preferably less than 1.0 wt.% water.

13. Method according to one of claims 1 to 12, **characterized in that** the components (a1), (a2) and (a3) - based on the total weight of the agent (a) - together have a weight proportion of at least 70.0 wt.%, preferably at least 80.0 wt.%, more preferably at least 90.0 wt.%, and most preferably at least 98.0 wt.%.

14. Method according to one of claims 1 to 13, **characterized in that** the agent (b) - based on the total weight of the agent (b) - contains 0 to 45 wt.%, preferably 5 to 40 wt.%, more preferably 10 to 35 wt.% and most preferably 15 to 35 wt.% water (b1).

15. Method according to one of claims 1 to 14, **characterized in that** the agent (b) contains one or more fatty components (b2) from the group of ester oils, C₁₂ -C₃₀ fatty alcohols, C₁₂ -C₃₀ fatty acid triglycerides, C₁₂ -C₃₀ fatty acid monoglycerides, C₁₂ -C₃₀ fatty acid diglycerides and/or hydrocarbons.

16. Method according to one of claims 1 to 15, **characterized in that** the agent (b) contains at least one fatty component (b2) from the group of esters of a C₆ -C₃₀ -alkanecarboxylic acid with an aliphatic C₂ -C₃₀ -monoalcohol.

17. Method according to one of claims 1 to 16, **characterized in that** the agent (b) contains at least one fatty component (b2) from the group consisting of stearic acid 2-ethylhexyl ester, isopropyl myristate, isononanoic acid C16-18 alkyl ester, 2-ethylhexyl palmitate, cetyl oleate, coconut fatty alcohol caprinate, coconut fatty alcohol caprylate, n-butyl stearate, oleylerucate, isopropyl palmitate, oleyloleate, lauric acid hexyl ester, myristyl myristate, cetearyl isononanoate and oleic acid decyl ester.

18. Method according to one of claims 1 to 17, **characterized in that** the agent (b) contains one or more fatty components in a total amount of 30 to 99 wt.%, preferably 40 to 97 wt.%, more preferably 50 to 95 wt.%, and most preferably 60 to 90 wt.%, based on the total weight of the agent (b).

19. Method according to one of claims 1 to 18, **characterized in that** the agent (b) contains at least one nonionic surfactant (b3).

20. Method according to one of claims 1 to 19, **characterized by** the
(3) preparation of an application mixture by mixing the agents (a) and (b) in a quantity ratio (a)/(b) of 1:2 to 1:200, preferably 1:5 to 1:100, more preferably 1:5 to 1:40, and most preferably 1:15 to 1:20.

21. Method according to one of claims 1 to 20, **characterized by**
(4) applying the application mixture to the keratinous material within a period of 1 to 120 minutes, preferably 1 to 60 minutes, more preferably 1 to 30 minutes, and most preferably 1 to 15 minutes after its preparation in step (3).

22. Method according to one of claims 1 to 21, **characterized by**
(5) allowing the application mixture applied in step (4) to act on the keratinous material for a period of 30 seconds to 15 minutes, preferably 30 seconds to 10 minutes, and most preferably 1 to 5 minutes.

23. Multi-component packaging unit (kit-of-parts) for coloring keratinous material, in particular human hair, comprising separately packaged
- a first container with an agent (a), wherein the agent (a) contains - based on the total weight of the agent (a) - 0 to 10 wt% water, and
(a1) at least one amino-functionalized silicone polymer, and
(a2) at least one coloring compound, and
(a3) optionally at least one solvent (a3), and
- a second container with an agent (b), wherein the agent (b) contains, based on the total weight of the agent (b):
(b1) 0 to 50 wt% water, and
(b2) at least one fatty component, and
(b3) optionally at least one nonionic surfactant.
wherein the ingredients (a1), (a2), (a3), (b1), (b2) and (b3) are defined in one of claims 1 to 19.

## Revendications

1. Procédé de coloration de matière kératinique, en particulier de cheveux humains, comprenant les étapes suivantes :
(1) fourniture d'un agent (a), l'agent (a) contenant, par rapport au poids total de l'agent (a), 0 à 10 % en poids d'eau et
(a1) au moins un polymère de silicone à fonction amino, et
(a2) au moins un composé colorant, et
(2) fourniture d'un agent (b), l'agent (b) contenant, par rapport au poids total de l'agent (b) :
(b1) 0 à 50 % en poids d'eau et
(b2) au moins un composant gras,
(3) préparation d'un mélange d'application par mélange des agents (a) et (b),
(4) application du mélange d'application préparé à l'étape (3) sur le matériau kératinique,
(5) laisser agir le mélange d'application appliqué à l'étape (4) sur le matériau kératinique, et
(6) rinçage du mélange d'application à l'eau.

2. Procédé selon la revendication 1, **caractérisé en ce que** l'agent (a) contient au moins un polymère de silicone aminofonctionnel (a1) avec au moins un groupe amino secondaire.

3. Procédé selon l'une des revendications 1 à 2, **caractérisé en ce que** l'agent (a) contient au moins un polymère de silicone aminofonctionnel (a1) qui comprend au moins une unité structurale de formule (Si-amino) 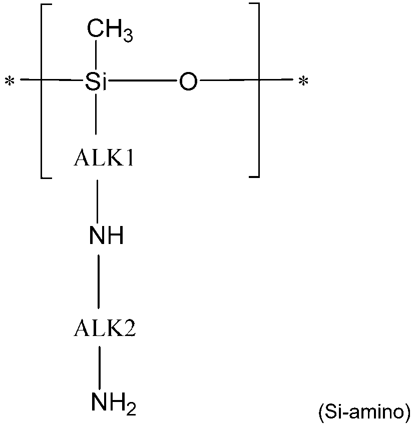 où
ALK1 et ALK2 représentent indépendamment l'un de l'autre un groupe alkylène divalent linéaire ou ramifié en C₁ -C₂₀.

4. Procédé selon l'une des revendications 1 à 3, **caractérisé en ce que** l'agent (a) contient au moins un polymère de silicone à fonction amino (a1) qui comprend des unités structurales de formule (Si-I) et de formule (Si-II)

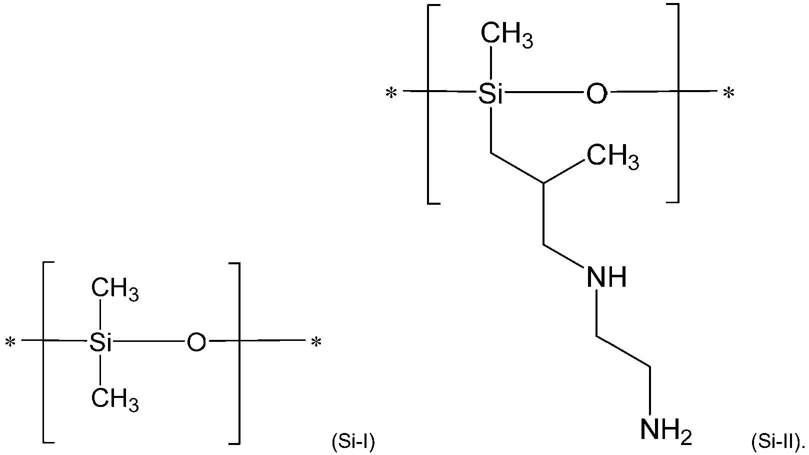

5. Procédé selon l'une des revendications 1 à 4, **caractérisé en ce que** l'agent (a) contient, par rapport au poids total de l'agent (a), un ou plusieurs polymères de silicone à fonction amine (a1) en une quantité totale de 2,0 à 95,0 % en poids, de préférence de 4,0 à 70,0 % en poids, de préférence de 6,0 à 50,0 % en poids et de manière particulièrement préférée de 8,0 à 20,0 % en poids.

6. Procédé selon l'une des revendications 1 à 5, **caractérisé en ce que** l'agent (a) contient au moins un composé colorant (a2) choisi parmi le groupe des pigments, des colorants directs, des colorants photochromiques et des colorants thermochromiques.

7. Procédé selon l'une des revendications 1 à 6, **caractérisé en ce que** l'agent (a) contient au moins un composé colorant (a2) du groupe des pigments inorganiques, qui est de préférence choisi dans le groupe des oxydes métalliques colorés, des hydroxydes métalliques, des hydrates d'oxydes métalliques, des silicates, sulfures métalliques, cyanures métalliques complexes, sulfates métalliques, pigments bronze et/ou pigments colorés à base de mica ou de micacé, qui sont recouverts d'au moins un oxyde métallique et/ou un oxychlorure métallique.

8. Procédé selon l'une des revendications 1 à 7, **caractérisé en ce que** l'agent (a) contient au moins un composé colorant (a2) choisi dans le groupe des pigments organiques, qui est de préférence choisi dans le groupe constitué par le carmin, la quinacridone, la phtalocyanine, le sorgho, les pigments bleus ayant les numéros d'index de couleur CI 42090, CI 69800, CI 69825, CI 73000, CI 74100, CI 74160, des pigments jaunes ayant les numéros d'index de couleur CI 11680, CI 11710, CI 15985, CI 19140, CI 20040, CI 21100, CI 21108, CI 47000, CI 47005, pigments verts portant les numéros d'index de couleur CI 61565, CI 61570, CI 74260, pigments orange portant les numéros d'index de couleur CI 11725, CI 15510, CI 45370, CI 71105, pigments rouges avec les numéros d'index de couleur CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 et/ou CI 75470.

9. Procédé selon l'une des revendications 1 à 8, **caractérisé en ce que** l'agent (a) contient, par rapport au poids total de l'agent (a), un ou plusieurs pigments en une quantité totale de 2,0 à 95,0 % en poids, de préférence de 4,0 à 70,0 % en poids, de préférence de 6,0 à 50,0 % en poids et de manière particulièrement préférée de 8,0 à 30,0 % en poids.

10. Procédé selon l'une des revendications 1 à 9, **caractérisé en ce que** l'agent (a) contient au moins un solvant (a3) choisi dans le groupe constitué par le 1,2-propylèneglycol, le 1,3-propylèneglycol, l'éthylèneglycol, le 1,2-butylèneglycol, le dipropylèneglycol, l'éthanol, l'isopropanol, éther monoéthylique de diéthylène glycol, glycérol, phénoxyéthanol et alcool benzylique, et de préférence tout particulièrement le 1,2-propylène glycol.

11. Procédé selon l'une des revendications 1 à 10, **caractérisé en ce que** l'agent (a)
- par rapport au poids total du produit (a) - un ou plusieurs solvants (a3) en une quantité totale de 1,0 à 95,0 % en poids, de préférence de 20,0 à 90,0 % en poids, de préférence de 40,0 à 85,0 % en poids et de manière particulièrement préférée de 60,0 à 85,0 % en poids.

12. Procédé selon l'une des revendications 1 à 11, **caractérisé en ce que** l'agent (a) contient, par rapport au poids total de l'agent (a), 0 à 5 % en poids, de préférence moins de 0 à 3 % en poids, et de manière particulièrement préférée moins de 1,0 % en poids d'eau.

13. Procédé selon l'une des revendications 1 à 12, **caractérisé en ce que** les composants (a1), (a2) et (a3) représentent ensemble, par rapport au poids total du produit (a), une proportion en poids d'au moins 70,0 % en poids, de préférence d'au moins 80,0 % en poids, de préférence d'au moins 90,0 % en poids et de manière particulièrement préférée d'au moins 98,0 % en poids.

14. Procédé selon l'une des revendications 1 à 13, **caractérisé en ce que** l'agent (b) contient, par rapport au poids total de l'agent (b), 0 à 45 % en poids, de préférence 5 à 40 % en poids, de préférence encore 10 à 35 % en poids et de manière particulièrement préférée 15 à 35 % en poids d'eau (b1).

15. Procédé selon l'une des revendications 1 à 14, **caractérisé en ce que** l'agent (b) contient un ou plusieurs composants gras (b2) choisis dans le groupe des huiles esters, des alcools gras en C₁₂ -C₃₀, des triglycérides d'acides gras en C₁₂ -C₃₀, des₁₂ -C₃₀ -acides gras, des C₁₂ -C₃₀ -diglycérides d'acides gras et/ou des hydrocarbures.

16. Procédé selon l'une des revendications 1 à 15, **caractérisé en ce que** l'agent (b) contient au moins un composant gras (b2) choisi dans le groupe des esters d'un acide alcane-carboxylique en C₆ - C₃₀ avec un monoalcool aliphatique en C₂ -C₃₀.

17. Procédé selon l'une des revendications 1 à 16, **caractérisé en ce que** l'agent (b) contient au moins un composant gras (b2) choisi dans le groupe constitué par l'ester 2-éthylhexylique de l'acide stéarique, le myristate d'isopropyle, l'ester alkylique en C16-18 de l'acide isononanoïque, le palmitate de 2-éthylhexyle, l' oléate de cétyle, caprinate d'alcool gras de coco, caprylate d'alcool gras de coco, stéarate de n-butyle, oléylérucate, palmitate d'isopropyle, oléate d'oléyle, hexylester d'acide laurique, myristate de myristyle, isononanoate de cétéaryle et décylester d'acide oléique.

18. Procédé selon l'une des revendications 1 à 17, **caractérisé en ce que** le produit (b) contient, par rapport au poids total du produit (b), un ou plusieurs composants gras dans une quantité totale de 30 à 99 % en poids, de préférence de 40 à 97 % en poids, de préférence encore de 50 à 95 % en poids et de manière particulièrement préférée de 60 à 90 % en poids.

19. Procédé selon l'une des revendications 1 à 18, **caractérisé en ce que** l'agent (b) contient au moins un tensioactif non ionique (b3).

20. Procédé selon l'une des revendications 1 à 19, **caractérisé par** les
(3) la préparation d'un mélange d'application par mélange des agents (a) et (b) dans un rapport quantitatif (a)/(b) de 1:2 à 1:200, de préférence de 1:5 à 1:100, de manière encore plus préférée de 1:5 à 1:40 et de manière tout à fait préférée de 1:15 à 1:20.

21. Procédé selon l'une des revendications 1 à 20, **caractérisé par** le
(4) application du mélange d'application sur le matériau kératinique dans un délai de 1 à 120 minutes, de préférence de 1 à 60 minutes, de préférence encore de 1 à 30 minutes et de manière particulièrement préférée de 1 à 15 minutes après sa préparation à l'étape (3).

22. Procédé selon l'une des revendications 1 à 21, **caractérisé par**
(5) laisser agir le mélange appliqué à l'étape (4) sur le matériau kératinique pendant une durée de 30 secondes à 15 minutes, de préférence de 30 secondes à 10 minutes, et de manière particulièrement préférée de 1 à 5 minutes.

23. Unité d'emballage à plusieurs composants (kit de pièces) pour la coloration de matière kératinique, en particulier de cheveux humains, comprenant des éléments confectionnés séparément les uns des autres
- un premier conteneur contenant un agent (a), l'agent (a) contenant, par rapport au poids total de l'agent (a), 0 à 10 % en poids d'eau, et
(a1) au moins un polymère de silicone à fonction amino, et
(a2) au moins un composé colorant, et
(a3) éventuellement au moins un solvant (a3), et
- un deuxième récipient contenant un agent (b), l'agent (b) contenant, par rapport au poids total de l'agent (b) :
(b1) 0 à 50 % en poids d'eau, et
(b2) au moins un composant gras, et
(b3) éventuellement au moins un tensioactif non ionique.
les composants (a1), (a2), (a3), (b1), (b2) et (b3) étant définis dans l'une des revendications 1 à 19.
